# EUROPEAN PATENT APPLICATION

(11) **EP 3 662 909 A1**
(43) Date of publication of application: **10.06.2020**
(21) Application number: 19218389.5
(22) Date of filing: 22.07.2016
(51) Int. Cl.: A61K 31/444, A61K 31/5383, A61K 31/437, A61K 38/05, A61K 31/18, A61K 31/27, A61K 31/352, A61K 31/4164, A61K 31/417, A61K 31/4245, A61K 31/454, A61P 35/00, A61K 38/06

(54) **GAMMA SECRETASE MODULATORS FOR THE TREATMENT OF IMMUNE SYSTEM DYSFUNCTION**

(30) Priority: 24.07.2015 US 201562196771 P; 27.07.2015 US 201562197469 P
(62) Divisional of application: 16831120.7
(71) Applicant: Oncotracker, Inc., West Hollywood, CA 90069 (US)
(72) Inventor: BERENSON, James Richard, Beverly Hills, CA California 90210 (US)
(74) Representative: Schiweck Weinzierl Koch Patentanwälte Partnerschaft mbB

(57) **Abstract**

The present invention provides methods of treating an immune system dysfunction or conditions associated with soluble BCMA using gamma secretase modulators or inhibitors in order to both restore immune function and improve the efficacy of therapies directed against BCMA present on the pathogenic B cell.

## Description

### CROSS REFERENCE TO RELATED APPLICATIONS

This application claims priority to U.S. Provisional Application No. 62/196,771 filed on July 24, 2015, and U.S. Provisional Application No. 62/197,469 filed on July 27, 2015, which are incorporated by reference herein in their entireties.

### BACKGROUND

### Technical Field

The invention generally relates to novel methods of treating or preventing immune system dysfunction. More particularly, the invention relates to methods of treating or preventing conditions or disorders associated with: immune system dysfunction; abnormal expression of B-cell maturation antigen (BCMA); and/or improving the efficacy of treatments for disorders characterized by immune system dysfunction or abnormal expression of BCMA through preventing BCMA shedding, with one or more gamma secretase modulators or a derivative thereof or other compounds that block the shedding of BCMA.

### Introduction

B cells are of pathogenic relevance and serve as a therapeutic target in generalized immunopathological diseases such as autoimmune disease, including but not limited to systemic lupus erythematosus (SLE), and hematological malignancies, including but not limited to multiple myeloma (MM).

Members of the tumor necrosis factor receptor (TNFR) superfamily and their ligands play a critical role in regulating the immune system and, in particular, controlling proliferation, differentiation and apoptosis of B cells. These include death receptors (CD95, TNFR1, DR4, DR5) that trigger apoptosis and receptors lacking a death domain (CD30, CD40, TNFR2), which promote cell proliferation, differentiation and survival. The TNF family members belonging to the B cell activating factor (BAFF; also named zTNF4, BLyS, TALL-1, THANK, TNFSF13B) ligand-receptor network have been closely linked to the homeostasis of B cells.

The BAFF ligand-receptor network controls the properties of B cells. BAFF can be recognized by the receptors BAFF-R (also named BR3), transmembrane activator and calcium modulator and cyclophilin ligand interactor (TACI), and B cell maturation (BCMA) antigen; these BAFF receptors are expressed predominantly on B cells in a temporal and maturation stage-specific fashion; BAFF signaling through each of these receptors mediates multiple functions of B cells. Failure of B cells to receive signals mediated by BAFF dramatically impairs B cell maturation and the maintenance of mature B cell populations. In contrast, excess BAFF signaling of B cells significantly increases the numbers of peripheral B cells and has been linked to chronic inflammatory disorders, autoimmune diseases and certain malignancies.

The present inventors have previously demonstrated that BCMA is present in the serum of patients having various B-cell malignancies, e.g., MM, chronic lymphocytic leukemia (CLL), Waldenstrom's macroglobulinemia (WM), and B cell non-Hodgkin's lymphomas (NHL). In addition, the present inventors have discovered that BCMA levels are increased in the serum of MM, CLL and WM patients compared to normal healthy subjects not afflicted with these cancers. The present inventors have also shown that the presence of BCMA in the serum contributes to the pathologies of various B cell malignancies, at least because serum BCMA can interact with and sequester BAFF, which can in turn reduce the activity of BCMA and other BAFF receptors on healthy B cells and disrupt normal immune function. Thus, there is a need in the art for a means to reduce levels of serum BCMA in order to prevent the sequestering of BAFF and possibly other B-cell ligands.

### BRIEF SUMMARY

The present invention generally provides novel and synergistic treatments for conditions and disorders associated with dysfunction of the immune system.

In various embodiments, the present invention contemplates methods of treating or preventing an immune system dysfunction in a subject comprising administering to the subject one or more gamma secretase modulators or a derivative thereof or other compounds that block the shedding of BCMA.

In various embodiments, the present invention contemplates methods of treating or preventing an immune system dysfunction, abnormal expression of BCMA, and/or shedding of BCMA from cells that express this protein in a subject comprising administering to the subject one or more gamma secretase modulators or a derivative thereof or other compounds that block the shedding of BCMA. In certain embodiments, the present invention contemplates methods of decreasing BCMA shedding from a plasma cell comprising administering one or more gamma secretase modulators, or a derivative thereof, to a subject having an immune system dysfunction, optionally wherein the immune system dysfunction is due to the expression of BCMA.

In various embodiments, the present invention contemplates methods of decreasing BCMA shedding from plasma and/or B-cells comprising administering to a subject having immune system dysfunction, one or more gamma secretase modulators or a derivative thereof.

In various embodiments, the present invention contemplates methods of increasing the efficacy of treatments for a subject being treated for a B cell condition or disorder or other disease associated with abnormal expression of BCMA comprising: administering to the subject one or more gamma secretase modulators or a derivative thereof in addition to the existing treatment being provided to the subject.

In certain embodiments, the present invention contemplates methods of increasing the efficacy of a therapy in a subject being treated for a B cell condition or disorder or other disease associated with abnormal expression of BCMA comprising: administering to the subject one or more gamma secretase modulators or a derivative thereof in addition to the existing treatment being provided to the subject.

In various embodiments, the immune system dysfunction is a B cell condition or disorder.

In a particular embodiment, the B cell condition or disorder is selected from the group consisting of: MM, CLL, WM, B cell non-Hodgkin's lymphoma, plasmacytoma, Hodgkins' lymphoma, follicular lymphomas, small non-cleaved cell lymphomas, endemic Burkitt's lymphoma, sporadic Burkitt's lymphoma, marginal zone lymphoma, extranodal mucosa-associated lymphoid tissue lymphoma, nodal monocytoid B cell lymphoma, splenic lymphoma, mantle cell lymphoma, large cell lymphoma, diffuse mixed cell lymphoma, immunoblastic lymphoma, primary mediastinal B cell lymphoma, pulmonary B cell angiocentric lymphoma, small lymphocytic lymphoma, B cell proliferations of uncertain malignant potential, lymphomatoid granulomatosis, post-transplant lymphoproliferative disorder, an immunoregulatory disorder, rheumatoid arthritis, myasthenia gravis, idiopathic thrombocytopenia purpura, anti-phospholipid syndrome, Chagas' disease, Grave's disease, Wegener's granulomatosis, poly-arteritis nodosa, Sjogren's syndrome, pemphigus vulgaris, scleroderma, multiple sclerosis, anti-phospholipid syndrome, ANCA associated vasculitis, Goodpasture's disease, Kawasaki disease, autoimmune hemolytic anemia, and rapidly progressive glomerulonephritis, heavy-chain disease, primary or immunocyte-associated amyloidosis, or monoclonal gammopathy of undetermined significance.

In one embodiment, the B cell condition or disorder is a B cell malignancy.

In a particular embodiment, the B cell condition or disorder is a plasma cell malignancy.

In one embodiment, the B cell condition or disorder is selected from the group consisting of: MM, WM, CLL, and B-cell non-Hodgkin's lymphoma.

In a certain embodiment, the B cell condition or disorder is MM

In a particular embodiment, the B cell condition or disorder is an autoimmune disease.

In a certain embodiment, the B cell condition or disorder is systemic lupus erythematosus.

In a particular embodiment, the B cell condition or disorder is rheumatoid arthritis.

In a further embodiment, the B cell condition or disorder is selected from the group consisting of: idiopathic thrombocytopenia purpura, myasthenia gravis, and autoimmune hemolytic anemia.

In a particular embodiment, the gamma secretase modulator is selected from the group consisting of: secretase inhibitor I (GSI I) Z-Leu-Leu-Norleucine; γ-secretase inhibitor II (GSI II); γ- secretase inhibitor III (GSI III), N-Benzyloxycarbonyl-Leuleucinal, N-(2-Naphthoyl)-Val- phenylalaninal; γ-secretase inhibitor III (GSI IV); γ-secretase inhibitor III (GSI V), N- Benzyloxycarbonyl-Leu- phenylalaninal; γ-secretase inhibitor III (GSI VI), 1-(S)-endo-N-(1,3,3)-Trimethylbicyclo[2.2.1]hept-2-yl)-4-fluorophenyl Sulfonamide; γ-secretase inhibitor III (GSI VII), Menthyloxycarbonyl-LL-CHO; γ-secretase inhibitor III (GSI IX), (DAPT), N- [N-(3,5- Difluorophenacetyl-L-alanyl)]-S-phenylglycine t- Butyl Ester; γ-secretase inhibitor X (GSI X), { 1 S-Benzyl-4R-[1-(1S-carbamoyl-2-phenethylcarbamoyl)-1S-3-methylbutylcarb-amoyl]-2R- hydroxy-5-phenylpentyl}carbamic Acid tert-butyl Ester; γ- secretase inhibitor XI (GSI XI), 7-Amino-4-chloro-3- methoxyisocoumarin; γ-secretase inhibitor XII (GSI XII), Z-Ile-Leu-CHO; γ-secretase inhibitor XIII (GSI XIII), Z-Tyr-Ile-Leu- CHO; γ-secretase inhibitor XIV (GSI XIV), Z-Cys(t-Bu)-Ile-Leu-CHO; γ-secretase inhibitor XVI (GSI XVI), N-[N-3,5-Difluorophenacetyl]-L- alanyl-S-phenylglycine Methyl Ester; γ- secretase inhibitor XVII (GSI XVII); γ-secretase inhibitor XIX (GSI XIX), benzo[e][1,4]diazepin-3-yl)-butyramide; γ-secretase inhibitor XX (GSI XX), (S,S)-2-[2-(3,5- Difluorophenyl)acetylamino]- N-(5-methyl-6-oxo-6,7- dihydro-5H- dibenzo[b,d]azepin-7- yl)propionamide; γ-secretase inhibitor XXI (GSI XXI), (S,S)-2-[2-(3,5-Difluorophenyl)-acetylamino]-N-(1-methyl-2-oxo-5-phenyl-2-,3-dihydro-1H-benzo[e][1,4]diazepin-3-yl)-propionamide; Gamma40 secretase inhibitor I, N-trans-3,5- Dimethoxycinnamoyl-Ile- leucinal; Gamma40 secretase inhibitor II, N-tert-Butyloxycarbonyl- Gly-Val-Valinal Isovaleryl-V V-Sta-A-Sta- OCH3; MK-0752 (Merck); MRK-003 (Merck); semagacestat/LY450139 (Eli Lilly); RO4929097; PF-03084,014; BMS-708163; MPC-7869 (γ-secretase modifier), YO-01027 (Dibenzazepine), Compound E ([(2S)-2-{[(3,5-Difluorophenyl)acetyl]amino}-N-[(3S)-1-methyl-2-oxo-5-phenyl-2,3-dihydro-1H-1,4-benzodiazepin-3-yl]propanamide], available from Alexis Biochemicals), LY411575 (Eli Lilly and Co.), L-685,458 (Sigma-Aldrich), BMS-289948 (4-chloro-N-(2,5-difluorophenyl)-N-((1R)-{4-fluoro-2-[3-(1H-imidazol-1-yl)propyl]phenyl}ethyl)benzenesulfonamide hydrochloride) and BMS-299897 (4-[2-((1R)-1-{[(4-chlorophenyl)sulfonyl]-2,5-difluoroanilino}ethyl)-5-fluorophenyl]butanoic acid) (Bristol Myers Squibb).

In a certain embodiment, the gamma secretase modulator or other compound that blocks BCMA shedding is intravenously administered to the subject.

In a certain embodiment, the gamma secretase modulator or other compound that blocks BCMA shedding is orally administered to the subject.

In one embodiment, the subject is being treated with or has been previously treated with radiation therapy, chemotherapy, transplantation, immunotherapy, hormone therapy, or photodynamic therapy.

In a particular embodiment, the subject is being treated with or has been previously treated for a B cell condition or disorder that targets BCMA on B cells.

In a particular embodiment, the subject has a hematological malignancy with detectable levels of serum BCMA

### BRIEF DESCRIPTION OF THE DRAWINGS

The accompanying drawings, which are incorporated in and constitute a part of this specification, illustrate several embodiments of the disclosed methods and compositions and together with the description, serve to explain the principles of the disclosed methods.

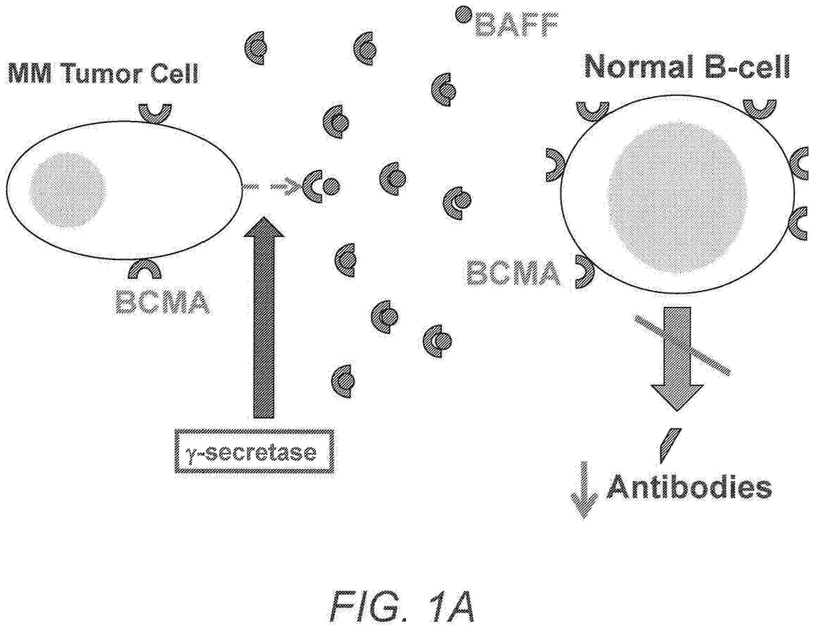

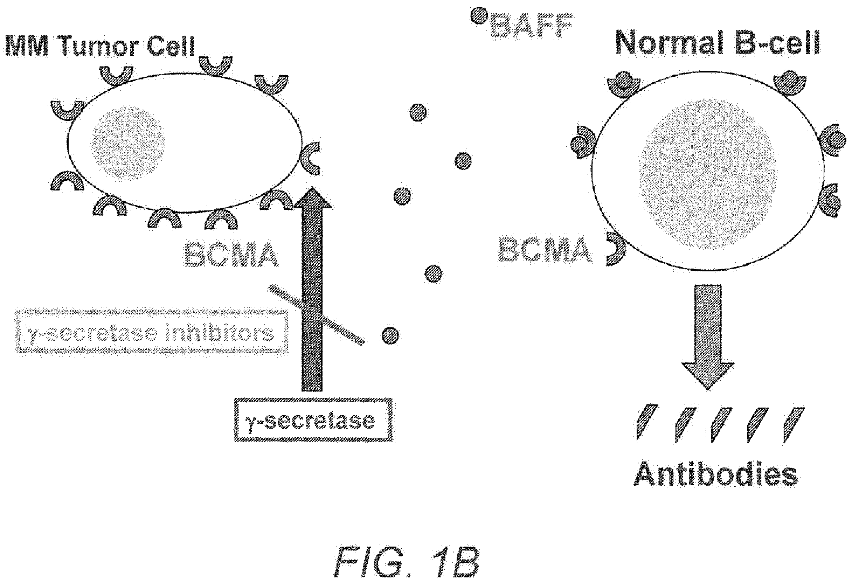

FIGS. 1A and 1B each show a diagram depicting a model whereby gamma secretase activity promotes BCMA shedding by MM cells.
FIG. 1A shows a scenario in which gamma secretase activity promotes BCMA (dark gray half circles) shedding from MM tumor cells (MM Tumor Cell; left). When BCMA shedding occurs, it liberates the extracellular portion of the BCMA from the MM tumor cell. The shed BCMA interacts with BAFF (light gray circles), preventing activation of intact BCMA by BAFF on normal B cells (Normal B-cell; right) which reduces antibody production from normal B-cells.
FIG. 1B shows a scenario where gamma secretase activity is decreased with a gamma secretase inhibitor. The reduction in gamma secretase activity reduces BCMA (dark gray half circles) shedding from MM tumor cells (MM Tumor Cell; left). Normally, BAFF (light gray circles) interacts with intact BCMA on nonmalignant B-cells (Normal B-cell; right), which leads to normal antibody production.

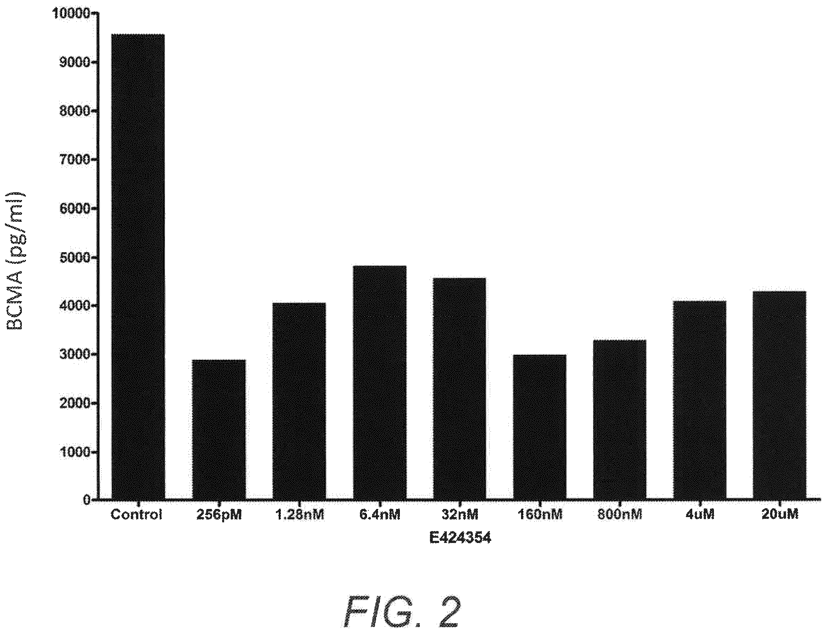

FIG. 2 shows a graph of supernatant BCMA levels in cultured LAGκ-1A tumor cells obtained from a human MM xenograft that was grown in severe combined immunodeficient mice and incubated with different concentrations of the gamma secretase inhibitor E424354 for 48 hours. Levels of supernatant BCMA are shown for cells cultured with (from left to right) 0 (control), 256 pM, 1.28 nM, 6.4 nM, 32 nM, 160 nM, 800 nM, 4 µM, and 20 µM concentrations of E424354.

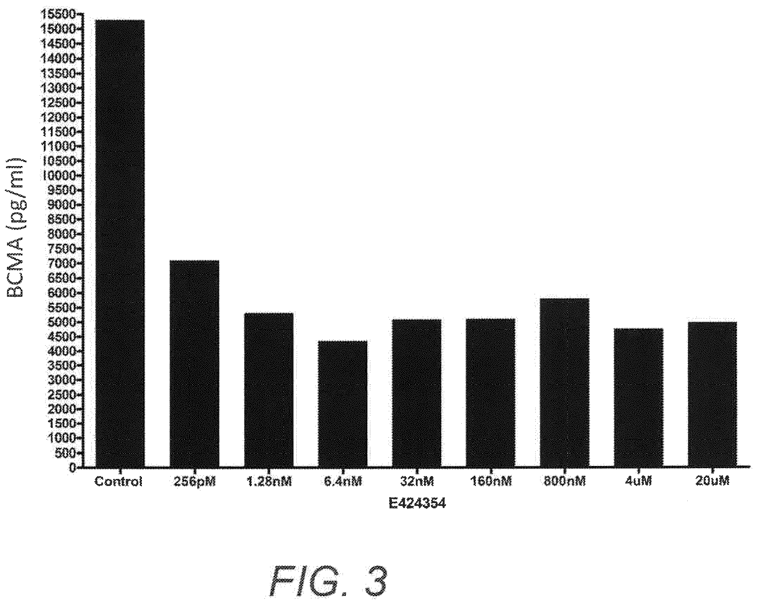

FIG. 3 shows a graph of supernatant BCMA levels in cultured LAGκ-1A tumor cells incubated with different concentrations of the gamma secretase inhibitor E424354 for 72 hours. Levels of supernatant BCMA are shown for cells cultured with (from left to right) 0 (control), 256 pM, 1.28 nM, 6.4 nM, 32 nM, 160 nM, 800 nM, 4 µM, and 20 µM concentrations of E424354.

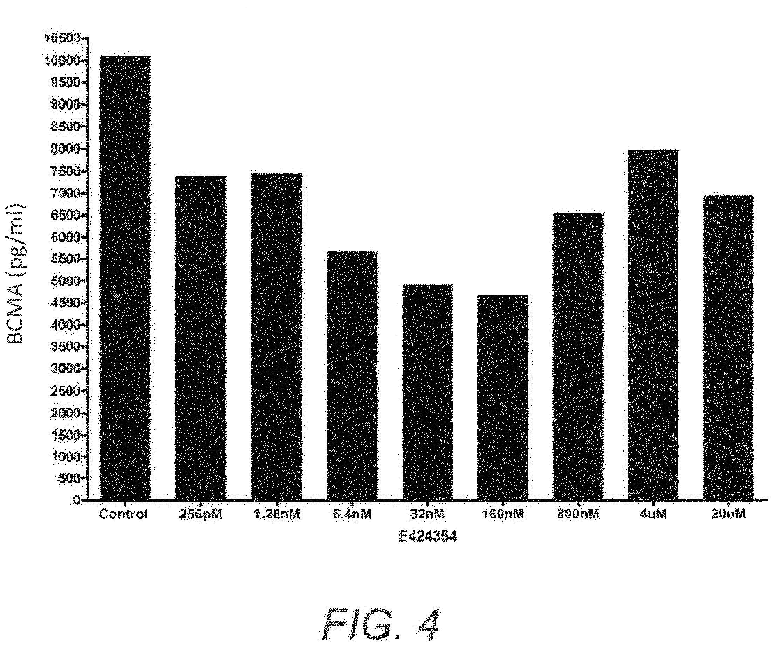

FIG. 4 shows a graph of supernatant BCMA levels in higher density cultured LAGκ-1A tumor cells incubated with different concentrations of the gamma secretase inhibitor E424354 for 48 hours. Levels of supernatant BCMA are shown for cells cultured with (from left to right) 0 (control), 256 pM, 1.28 nM, 6.4 nM, 32 nM, 160 nM, 800 nM, 4 µM, and 20 µM concentrations of E424354.

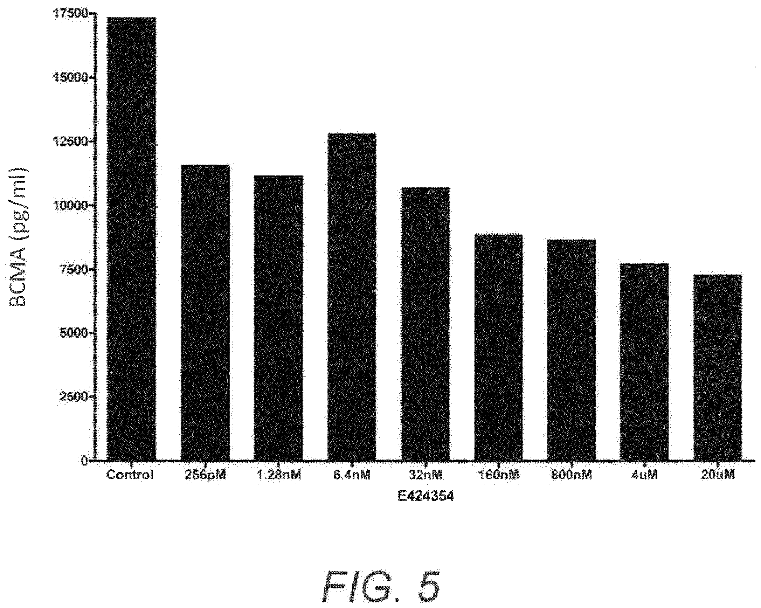

FIG. 5 shows a graph of supernatant BCMA levels LAGκ-1A tumor cells cultured at a higher cellular density and incubated with different concentrations of the gamma secretase inhibitor E424354 for 72 hours. Levels of supernatant BCMA are shown for cells cultured with (from left to right) 0 (control), 256 pM, 1.28 nM, 6.4 nM, 32 nM, 160 nM, 800 nM, 4 µM, and 20 µM concentrations of E424354.

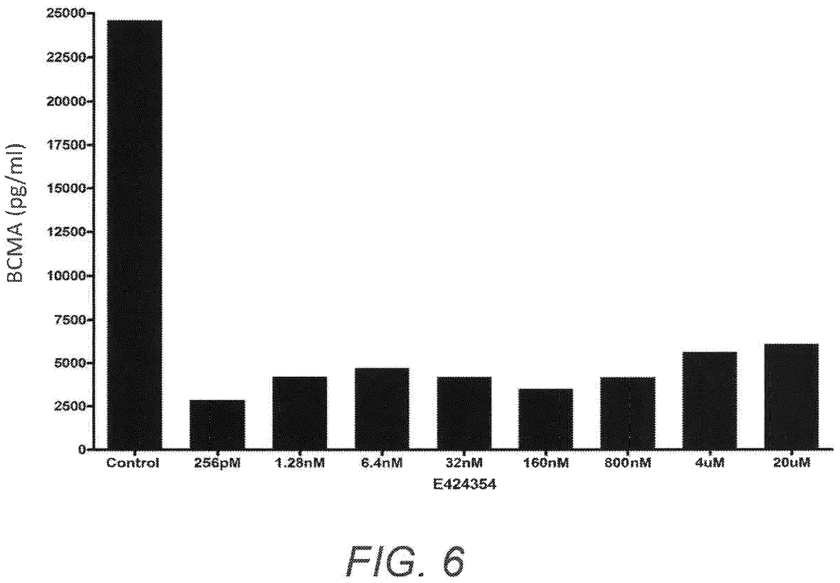

FIG. 6 shows a graph of supernatant BCMA levels in LAGκ-1A tumor cells cultured at a higher density and incubated with different concentrations of the gamma secretase inhibitor E424354 for 5 days. Levels of supernatant BCMA are shown for cells cultured with (from left to right) 0 (control), 256 pM, 1.28 nM, 6.4 nM, 32 nM, 160 nM, 800 nM, 4 µM, and 20 µM concentrations of E424354.

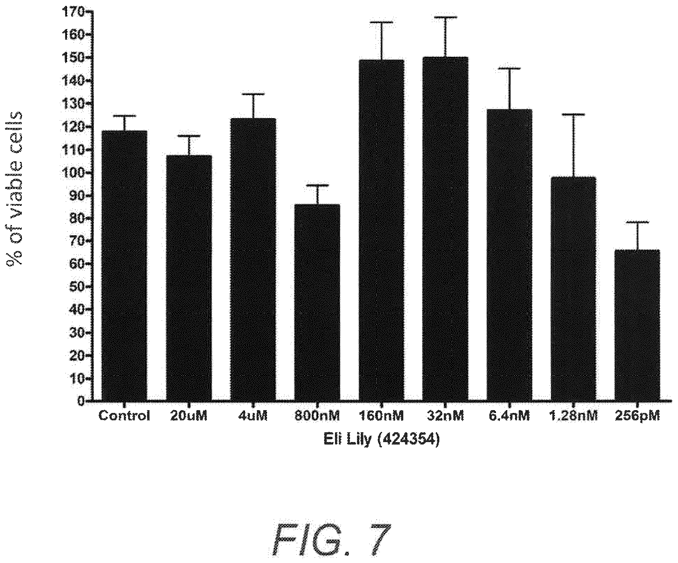

FIG. 7 shows a graph displaying percentage of viable LAGκ-1A tumor cells as determined by MTS assay following incubation with different concentrations of the gamma secretase inhibitor E424354 for 24 hours. The percentages of viable cells are shown for cells cultured with (from left to right) 0 (control), 20 µM, 4 µM, 800 nM, 160 nM, 32 nM, 6.4 nM, 1.28 nM, and 256 pM concentrations ofE424354.

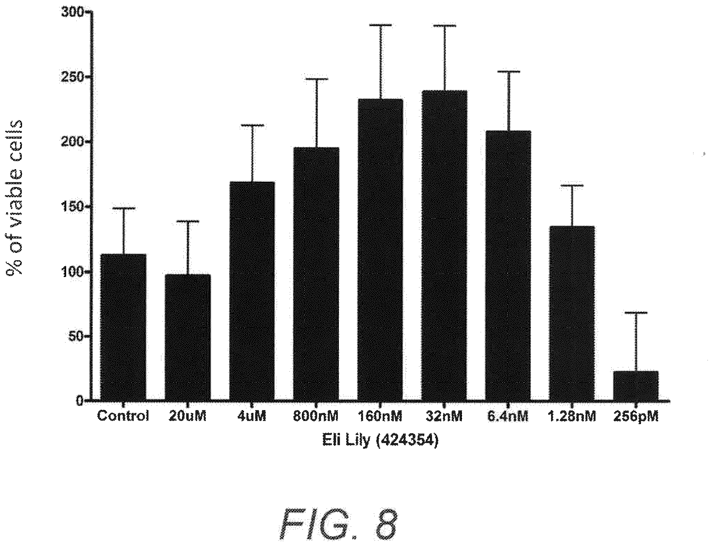

FIG. 8 shows a graph displaying percentage of viable LAGκ-1A tumor cells as determined by MTS assay following incubation with different concentrations of the gamma secretase inhibitor E424354 for 48 hours. The percentages of viable cells are shown for cells cultured with (from left to right) 0 (control), 20 µM, 4 µM, 800 nM, 160 nM, 32 nM, 6.4 nM, 1.28 nM, and 256 pM concentrations ofE424354.

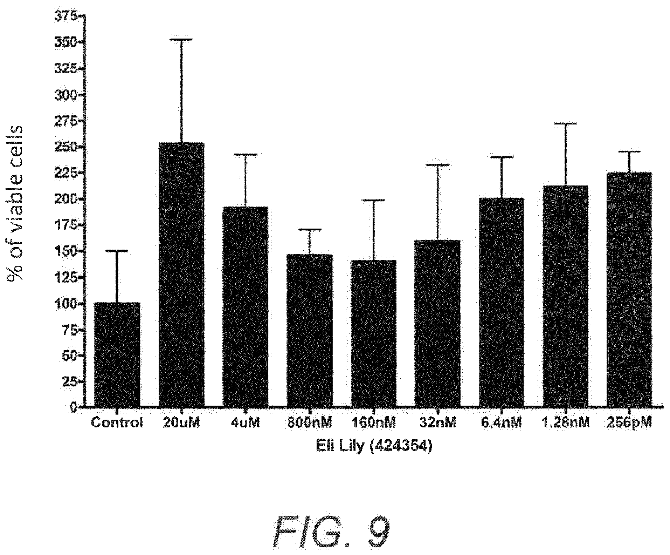

FIG. 9 shows a graph displaying percentage of viable LAGκ-1A tumor cells as determined using the MTS assay following incubation with different concentrations of the gamma secretase inhibitor E424354 for 72 hours. The percentages of viable cells are shown for cells cultured with (from left to right) 0 (control, 20 µM, 4 µM, 800 nM, 160 nM, 32 nM, 6.4 nM, 1.28 nM, and 256 pM concentrations of E424354.

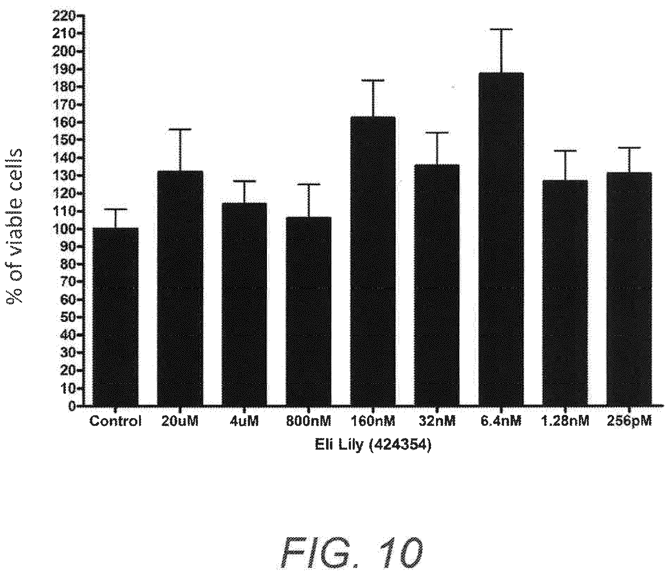

FIG. 10 shows a graph displaying percentage of viable LAGκ-1A tumor cells as determined with an MTS assay following incubation with different concentrations of the gamma secretase inhibitor E424354 for 5 days. The percentages of viable cells are shown for cells cultured with (from left to right) 0 (control), 20 µM, 4 µM, 800 nM, 160 nM, 32 nM, 6.4 nM, 1.28 nM, and 256 pM concentrations of E424354.

### DETAILED DESCRIPTION

### A. Overview

B-cell maturation antigen (BCMA) is expressed on the surface of plasma cells and regulates their survival. BCMA is detected in the serum of patients with several different types of B cell malignancies and levels of BCMA in the serum correlated with disease activity and overall survival of these patients. Gamma secretase is involved in the cleavage of membrane bound BCMA and shedding of the BCMA's extracellular domain into the serum. Without wishing to be bound by any particular theory, it is contemplated that gamma secretase modulators will decrease, prevent or block BCMA cleavage and its shedding, thereby preventing the associated immune system dysfunction, e.g., autoimmune disease or hematological malignancy. Furthermore, it is contemplated that use of gamma secretase inhibitors to prevent BCMA shedding will also increase the effectiveness of therapies that now in clinical development that target pathological B cells expressing BCMA

Without being bound by theory, particular embodiments contemplate a model whereby gamma secretase activity promotes BCMA shedding from pathogenic B cells in a subject, which in turn contributes to immune system dysfunction in the subject (see FIG. 1A). According to this model, gamma secretase activity in pathogenic B cells (*e.g.,* multiple myeloma tumor cells) promotes BCMA shedding which releases the extracellular portion of the BCMA (referred to herein as soluble BCMA or serum BCMA) from B cells. The soluble BCMA can sequester circulating BCMA ligands, e.g., BAFF, which in turn can prevent or reduce the interaction of BCMA ligands with BCMA on normal, non-pathogenic B cells. According to this model, the reduced interactions between BCMA ligands, e.g., BAFF, and BCMA present on the surface of normal, non-pathogenic, B cells disrupts normal immune system functioning, such as production of normal, polyclonal antibodies.

Without being bound by theory, certain embodiments contemplate a model whereby reducing gamma secretase activity in a subject, e.g., through administration of a gamma secretase inhibitor, prevents BCMA shedding from pathogenic B cells. This allows the ligands BAFF and APRIL to remain unbound since soluble BCMA levels are now reduced; and, as a result, allow them to carry out their normal functions to stimulate B cells to produce antibodies. Thus, treatment with a gamma secretase inhibitor should treat or prevent immune system dysfunction in the subject (see FIG. 1B). According to this model, the reduction in gamma secretase activity prevents or reduces BCMA shedding by pathogenic B cells, *e.g.* MM tumor cells, which in turn reduces the amount of soluble BCMA. Certain embodiments contemplate that reducing the amount of soluble BCMA restores the normal interactions between BCMA ligands, *e.g.,* BAFF, and BCMA present on the surface of normal, non-pathogenic B cells, thereby restoring normal immune function, *e.g.,* production of normal, polyclonal antibodies (see FIG. 1B).

Without being bound by theory, some embodiments contemplate a model whereby reducing gamma secretase activity in pathogenic B cells (*e.g.*, multiple myeloma tumor cells) will reduce the amount of BCMA shedding by the pathogenic B cells and thereby increase the amount of BCMA expressed by pathogenic B cells (see FIG. 1B). In certain embodiments, this model contemplates that reducing gamma secretase activity in pathogenic B cells enhances or improves the efficacy of a therapeutic agent that binds to BCMA and/or binds to cells that express this cell surface receptor that is administered for the treatment of a B cell condition or disorder. Therapeutic agents that bind to BCMA and/or cell expressing BCMA include, but are not limited to, therapeutic anti-BCMA antibodies or fragments thereof, anti-BCMA antibody-drug conjugates, and chimeric antigen receptor T cells (CAR-T cells) that bind to BCMA

In various embodiments, the present invention contemplates, in part, immune system therapies comprising of one or more gamma secretase modulators or other compounds that prevent shedding of BCMA

All publications, patents and patent applications cited herein are hereby incorporated by reference in their entirety.

### B. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred embodiments of compositions, methods and materials are described herein. For the purposes of the present invention, the following terms are defined below.

The articles "a," "an," and "the" are used herein to refer to one or to more than one (*i.e.,* to at least one) of the grammatical object of the article. By way of example, "an element" means one element or more than one element.

As used herein, the term "about" or "approximately" refers to a quantity, level, value, number, frequency, percentage, dimension, size, amount, weight or length that varies by as much as 30, 25, 20, 25, 10, 9, 8, 7, 6, 5, 4, 3, 2, or 1 % to a reference quantity, level, value, number, frequency, percentage, dimension, size, amount, weight or length. In particular embodiments, the terms "about" or "approximately" when preceding a numerical value indicate the value plus or minus a range of 15%, 10%, 5%, or 1%.

Throughout this specification, unless the context requires otherwise, the words "comprise," "comprises," and "comprising" will be understood to imply the inclusion of a stated step or element or group of steps or elements but not the exclusion of any other step or element or group of steps or elements. By "consisting of' is meant including, and limited to, whatever follows the phrase "consisting of." Thus, the phrase "consisting of' indicates that the listed elements are required or mandatory, and that no other elements may be present. By "consisting essentially of' is meant including any elements listed after the phrase, and limited to other elements that do not interfere with or contribute to the activity or action specified in the disclosure for the listed elements. Thus, the phrase "consisting essentially of' indicates that the listed elements are required or mandatory, but that no other elements are optional and may or may not be present depending upon whether or not they affect the activity or action of the listed elements

Reference throughout this specification to "one embodiment," "an embodiment," "another embodiment," "a particular embodiment," "a related embodiment," "a certain embodiment," "an additional embodiment," or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments.

The terms "treating," "treatment," and the like are used herein to generally mean obtaining a desired pharmacologic and/or physiologic effect. The effect may be prophylactic in terms of completely or partially preventing a disease and/or may be therapeutic in terms of a partial or complete cure for a disease and/or adverse effect attributable to the disease. "Treatment" as used herein covers any treatment of a disease in a mammal, and includes: preventing the disease from occurring in a subject which may be predisposed to the disease but has not yet been diagnosed as having it; inhibiting the disease, *i.e.,* arresting its development; or relieving the disease, *i.e.,* causing regression of the disease. The therapeutic agent may be administered before, during or after the onset of disease or injury. The treatment of ongoing disease, where the treatment stabilizes or reduces the undesirable clinical symptoms of the patient, is of particular interest.

As used herein, the phrase "ameliorating at least one symptom of' refers to decreasing one or more symptoms of the disease or condition for which the subject is being treated. In particular embodiments, the disease or condition being treated is a B cell condition or disorder, wherein the one or more symptoms ameliorated include, but are not limited to, weakness, fatigue, shortness of breath, easy bruising and bleeding, frequent infections, enlarged lymph nodes, distended or painful abdomen (due to enlarged abdominal organs), bone or joint pain, fractures, unplanned weight loss, poor appetite, night sweats, persistent mild fever, and decreased urination (due to impaired kidney function). In particular embodiments, the disease or condition being treated is a multiple myeloma, wherein the one or more symptoms ameliorated include bone pain.

As used herein, "prevent," and similar words such as "prevented," "preventing" *etc.,* indicate an approach for preventing, inhibiting, or reducing the likelihood of the occurrence or recurrence of, a disease or condition. It also refers to delaying the onset or recurrence of a disease or condition or delaying the occurrence or recurrence of the symptoms of a disease or condition. As used herein, "prevention" and similar words also include reducing the intensity, effect, symptoms and/or burden of a disease or condition prior to onset or recurrence of the disease or condition.

As used herein, the term "amount" refers to "an amount effective" or "an effective amount" of cells sufficient to achieve a beneficial or desired prophylactic or therapeutic result, including clinical results. In one embodiment, an effect amount refers to the amount of a gamma secretase inhibitor or a derivative thereof sufficient to prevent, ameliorate one symptom of, or treat a disease, e.g., a B cell condition or disorder contemplated herein.

A "prophylactically effective amount" refers to an amount of a gamma secretase inhibitor or a derivative thereof effective to achieve the desired prophylactic result. Typically but not necessarily, since a prophylactic dose is used in subjects prior to or at an earlier stage of disease, the prophylactically effective amount is less than the therapeutically effective amount.

A "therapeutically effective amount" of a gamma secretase inhibitor or a derivative thereof may vary according to factors such as the disease state, age, sex, and weight of the individual, and the agent to elicit a desired response in the individual. A therapeutically effective amount is also one in which any toxic or detrimental effects of the agent are outweighed by the therapeutically beneficial effects. The term "therapeutically effective amount" includes an amount that is effective to "treat" a subject (*e.g.,* a patient).

As used herein, the terms "conditions sufficient," or "under conditions sufficient," refer to the conditions for treating the subject, with one or more agents or compositions contemplated herein. In one embodiment, "conditions sufficient" include administering a sufficient amount, e.g., an effective amount of a gamma secretase inhibitor or a derivative thereof to a subject in need thereof.

As used herein, the terms "promoting," "enhancing," "stimulating," or "increasing" generally refer to the ability of compositions contemplated herein to produce or cause a greater physiological response (*i.e.,* measurable downstream effect), as compared to the response caused by either vehicle or a control molecule/composition. One such measurable physiological response includes, without limitation, increased cell killing and/or tumor reduction, increased survival, increased treatment efficacy compared to normal, untreated, or control-treated subjects. The physiological response may be increased by at least 5%, 10%, 20%, 30%, 40%, 50%, 60%, 70%, 80%, 90%, 100%, 125%, 150%, 175%, 200%, or greater compared to the response measured in normal, untreated, or control-treated subjects. An "increased" or "enhanced" response or property is typically "statistically significant" , and may include an increase that is 1.1, 1.2, 1.5, 2, 3, 4, 5, 6, 7, 8, 9, 10, 15, 20, 30 or more times (*e.g.,* 500, 1000 times) (including all integers and decimal points in between and above 1, *e.g.,* 1.5, 1.6, 1.7. 1.8, *etc.*) that produced by normal, untreated, or control-treated subjects.

As used herein, the terms "decrease" or "lower," or "lessen," or "reduce," or "abate" refers generally to the ability of compositions contemplated to produce or cause a lesser physiological response (*i.e.,* downstream effects), as compared to the response caused by either vehicle or a control molecule/composition, *e.g.,* decreased BCMA shedding. The physiological response, *e.g.,* BCMA shedding, may be decreased by at least 5%, 10%, 20%, 30%, 40%, 50%, 60%, 70%, 80%, 90%, 100%, 125%, 150%, 175%, 200%, or greater compared to the response measured in normal, untreated, or control-treated subjects. A "decrease" or "reduced" response is typically a "statistically significant" response, and may include an decrease that is 1.1, 1.2, 1.5, 2, 3, 4, 5, 6, 7, 8, 9, 10, 15, 20, 30 or more times (*e.g.,* 500, 1000 times) (including all integers and decimal points in between and above 1, *e.g.,* 1.5, 1.6, 1.7. 1.8, *etc.*) the response produced by normal, untreated, or control-treated subject.

"Immune system dysfunction" means any disorder or condition of the immune system. In various embodiments, immune system dysfunction refers to disorders or conditions of the immune system associated with abnormal or pathologic soluble BCMA levels in the serum or plasma of a subject. In various embodiments, immune system dysfunction refers to disorders or conditions of the immune system associated with abnormal or pathologic BCMA expression levels on immune cells in a subject. Immune system dysfunctions include both B cell and T cell disorders. Without wishing to be bound by any particular theory, it is contemplated that there are many different immune system dysfunctions that can be treated with the compositions and methods contemplated herein because altered the level of soluble BCMA expression levels would alter the effects of its ligands BAFF and APRIL on B cells and T cells. In particular embodiments, the dysfunction of the immune system occurs among patients with B cell conditions or disorders.

"Hematological malignancy" is a type of cancer that affects blood, bone marrow or lymph nodes. Hematological malignancies may derive from either of the two major blood cell lineages: myeloid or lymphoid cell lines. The myeloid cell line normally produces granulocytes, erythrocytes, thrombocytes, macrophages, and mast cells, whereas the lymphoid cell lines produce B-cells, T-cells, natural killer cells, and plasma cells. Lymphomas, lymphocytic leukemias and myeloma are from the lymphoid cell line. Illustrative examples of hematological malignancies that can be treated with compositions contemplated herein include myelomas, leukemias and lymphomas. Other illustrative examples of hematological malignancies that are suitable for treatment in particular embodiments of the methods contemplated herein include, but are not limited to, MM, WM, acute myeloid leukemia (AML), chronic myeloid leukemia (CML), acute lymphatic leukemia (ALL), CLL, Hodgkin's disease, non-Hodgkin lymphoma, myelodysplastic syndrome (MDS) or myeloproliferative diseases. Usually, hematological malignancies do not form solid tumors.

A "subject," "subject in need of treatment," "subject in need thereof," "individual," or "patient" as used herein, includes any animal that exhibits a symptom of a disease, disorder, or condition that can be treated with compositions contemplated herein. In particular embodiments, the disease, disorder, or condition relates to a hematological malignancy, *e.g.,* multiple myeloma. Suitable subjects include laboratory animals (such as mouse, rat, rabbit, or guinea pig), farm animals (such as horses, cows, sheep, pigs), and domestic animals or pets (such as a cat or dog). In particular embodiments, the subject is a mammal. In certain embodiments, the subject is a non-human primate and, in preferred embodiments, the subject is a human.

The term "relapse" refers to the diagnosis of return, or signs and symptoms of return, of a cancer after a period of improvement or remission.

"Remission," also known as "clinical remission," includes both partial and complete remission. In partial remission, some, but not all, signs and symptoms of cancer have disappeared. In complete remission, all signs and symptoms of cancer have disappeared, although cancer still may be in the body.

"Refractory" refers to a cancer that is resistant to, or non-responsive to, therapy with a particular therapeutic agent. A cancer can be refractory from the onset of treatment (*i.e.,* non-responsive to initial exposure to the therapeutic agent), or as a result of developing resistance to the therapeutic agent, either over the course of a first treatment period or during a subsequent treatment period.

The term "agent" refers to a natural or synthetic polypeptide, polynucleotide, carbohydrate, fatty acid, chemical compound, or small organic molecule. In particular embodiments, the agent comprises a gamma secretase modulator.

### C. Gamma Secretase Modulators

In various embodiments, a subject is administered one or more gamma secretase modulators, e.g., gamma secretase inhibitor, or a derivative thereof to prevent, treat, or ameliorate at least one symptom of, an immune system dysfunction, a B cell condition or disorder and/or to prevent BCMA shedding on a B cell.

"Gamma secretase modulator" refers to a compound or agent that modulates, increases, or decreases gamma secretase activity. Gamma secretase modulators include gamma secretase inhibitors, which decrease or reduce gamma secretase activity.

Numerous gamma secretase inhibitors have been described. See, for example, U.S. Pat. Nos. 6,756,511; 6,890,956; 6,984,626; 7,049,296; 7,101,895; 7,138,400; 7,144,910; 7,183,303; Bihel *et al.* 2004; Best *et al.* 2006; Davies *et al.* 2007; El-Gendy and Adejare 2004; Laras *et al.* 2005; McLendon *et al.* 2000; Prasad *et al.* 2007; Shearman *et al.* 2000; and Tomita *et al.* 2004; each of which is incorporated herein by reference, in its entirety.

Illustrative examples of gamma secretase modulators and inhibitors include, but are not limited to: secretase inhibitor I (GSI I) Z-Leu-Leu-Norleucine; γ-secretase inhibitor II (GSI II); γ- secretase inhibitor III (GSI III), N-Benzyloxycarbonyl-Leu- leucinal, N-(2-Naphthoyl)-Val- phenylalaninal; γ-secretase inhibitor III (GSI IV); γ-secretase inhibitor III (GSI V), N- Benzyloxycarbonyl-Leu- phenylalaninal; γ-secretase inhibitor III (GSI VI), 1-(S)-endo-N- (1,3,3)- Trimethylbicyclo[2.2.1]hept-2-yl)-4-fluorophenyl Sulfonamide; γ-secretase inhibitor III (GSI VII), Menthyloxycarbonyl-LL-CHO; γ-secretase inhibitor III (GSI IX), (DAPT), N- [N-(3,5- Difluorophenacetyl-L- alanyl)]-S-phenylglycine t- Butyl Ester; γ-secretase inhibitor X (GSI X), {1S-Benzyl-4R-[1-(1S-carbamoyl-2-phenethylcarbamoyl)-1S-3-methylbutylcarb-amoyl]-2R- hydroxy-5-phenylpentyl}carbamic Acid tert-butyl Ester; γ- secretase inhibitor XI (GSI XI), 7-Amino-4-chloro-3- methoxyisocoumarin; γ-secretase inhibitor XII (GSI XII), Z-Ile-Leu-CHO; γ-secretase inhibitor XIII (GSI XIII), Z-Tyr-Ile-Leu- CHO; γ-secretase inhibitor XIV (GSI XIV), Z-Cys(t-Bu)-Ile-Leu-CHO; γ-secretase inhibitor XVI (GSI XVI), N-[N-3,5-Difluorophenacetyl]-L- alanyl-S-phenylglycine Methyl Ester; γ- secretase inhibitor XVII (GSI XVII); γ-secretase inhibitor XIX (GSI XIX), benzo[e][1,4]diazepin-3-yl)-butyramide; γ-secretase inhibitor XX (GSI XX), (S,S)-2-[2-(3,5- Difluorophenyl)acetylamino]- N-(5-methyl-6-oxo-6,7-dihydro-5H-dibenzo[b,d]azepin-7- yl)propionamide; γ-secretase inhibitor XXI (GSI XXI), (S,S)-2-[2-(3,5-Difluorophenyl)-acetylamino]-N-(1-methyl-2-oxo-5-phenyl-2-,3-dihydro-1H-benzo[e][1,4]diazepin-3-yl)-propionamide; Gamma40 secretase inhibitor I, N-trans-3,5- Dimethoxycinnamoyl-Ile- leucinal; Gamma40 secretase inhibitor II, N-tert-Butyloxycarbonyl- Gly-Val-Valinal Isovaleryl-V V-Sta-A-Sta- OCH3; MK-0752 (Merck); MRK-003 (Merck); semagacestat/LY450139 (Eli Lilly); RO4929097; PF-03084,014; BMS-708163; MPC-7869 (γ-secretase modifier), YO-01027 (Dibenzazepine), Compound E ([(2S)-2-{[(3,5-Difluorophenyl)acetyl]amino}-N-[(3S)-1-methyl-2-oxo-5-phenyl-2,3-dihydro-1H-1,4-benzodiazepin-3-yl]propanamide], available from Alexis Biochemicals), LY411575 (Eli Lilly and Co.), L-685,458 (Sigma-Aldrich), BMS-289948 (4-chloro-N-(2,5-difluorophenyl)-N-((1R)-{4-fluoro-2-[3-(1H-imidazol-1-yl)propyl]phenyl}ethyl)benzenesulfonamide hydrochloride) and BMS-299897 (4-[2-((1R)-1-{[(4-chlorophenyl)sulfonyl]-2,5-difluoroanilino}ethyl)-5-fluorophenyl]butanoic acid) (Bristol Myers Squibb).

In particular embodiments, a gamma secretase modulator or inhibitor suitable for use in the compositions and methods contemplated herein includes any agent that directly or indirectly inhibits the cleavage of BCMA by gamma secretase or a gamma secretase activity. In certain embodiments, a gamma secretase inhibitor may decrease gamma secretase activity in a cell about 5%, about 10%, about 15%, about 20%, about 25%, about 30%, about 20%, about 25%, about 30%, about 35%, about 40%, about 45%, about 50%, about 55%, about 60%, about 65%, about 70%, about 75%, about 80%, about 85%, about 90%, about 95%, about 100%, compared to a cell that has not been contacted by the gamma secretase inhibitor. In certain embodiments, a gamma secretase modulator or inhibitor may decrease gamma secretase levels in a cell about 5%, about 10%, about 15%, about 20%, about 25%, about 30%, about 20%, about 25%, about 30%, about 35%, about 40%, about 45%, about 50%, about 55%, about 60%, about 65%, about 70%, about 75%, about 80%, about 85%, about 90%, about 95%, about 100%, compared to a cell that has not been contacted by the gamma secretase modulator or inhibitor.

In particular embodiments, a gamma secretase modulator or inhibitor may decrease BCMA cleavage or shedding about 5%, about 10%, about 15%, about 20%, about 25%, about 30%, about 20%, about 25%, about 30%, about 35%, about 40%, about 45%, about 50%, about 55%, about 60%, about 65%, about 70%, about 75%, about 80%, about 85%, about 90%, about 95%, about 100%, compared to the level of in a cell or cells that have not been contacted by the gamma secretase modulator or inhibitor.

In particular embodiments, a gamma secretase modulator or inhibitor may decrease BCMA detected in the serum of a subject administered one or more gamma secretase inhibitors by about 5%, about 10%, about 15%, about 20%, about 25%, about 30%, about 20%, about 25%, about 30%, about 35%, about 40%, about 45%, about 50%, about 55%, about 60%, about 65%, about 70%, about 75%, about 80%, about 85%, about 90%, about 95%, about 100%, compared to the serum of a subject that has not been administered one or more gamma secretase modulators or inhibitors.

In particular embodiments, administering a gamma secretase modulator or inhibitor to a subject may increase the interaction between BAFF and BCMA on a non-pathogenic B cell of the subject by about 5%, about 10%, about 15%, about 20%, about 25%, about 30%, about 20%, about 25%, about 30%, about 35%, about 40%, about 45%, about 50%, about 55%, about 60%, about 65%, about 70%, about 75%, about 80%, about 85%, about 90%, about 95%, about 100%, about 1.5-fold, about 2-fold, about 2.5-fold, about 3-fold, about 4-fold, about 5-fold, about 10-fold, about 20-fold, about 30-fold, about 40-fold, about 50-fold, about 100-fold, or greater than 100-fold (including all ranges and values in-between) as compared to the serum of a subject that has not been administered one or more gamma secretase modulators or inhibitors.

In certain embodiments, a gamma secretase modulator or inhibitor may decrease BCMA cleavage or shedding in a pathogenic B cell. A "pathogenic B cell", as used herein, refers to a B cell that is known or suspected to contribute to at least one sign or symptom of a B cell condition or disorder. In some embodiments, the pathogenic B cell produces auto antibodies. In certain embodiments, the pathogenic B cell is a type of cancer cell. In some embodiments, the B cell is a MM cancer cell, a CLL cancer cell, a WM cancer cell, a non-Hodgkin's lymphoma cancer cell, or a Hodgkin's lymphoma cancer cell.

In particular embodiments, a gamma secretase modulator or inhibitor may increase an amount of BCMA detected in a pathogenic B cell by about 5%, about 10%, about 15%, about 20%, about 25%, about 30%, about 20%, about 25%, about 30%, about 35%, about 40%, about 45%, about 50%, about 55%, about 60%, about 65%, about 70%, about 75%, about 80%, about 85%, about 90%, about 95%, about 100%, about 1.5-fold, about 2-fold, about 2.5-fold, about 3-fold, about 4-fold, about 5-fold, about 10-fold, about 20-fold, about 30-fold, about 40-fold, about 50-fold, about 100-fold, or greater than 100-fold (including all ranges and values in-between)compared to the serum of a subject that has not been administered one or more gamma secretase modulators or inhibitors.

### D. Pharmaceutical Compositions and Formulations

Compositions (*i.e.,* medicaments) contemplated herein include, but are not limited to pharmaceutical compositions. A "pharmaceutical composition" refers to a formulation of a composition with one or more pharmaceutically acceptable carriers, diluents or excipients generally accepted in the art for the delivery of a compound or drug to a mammal, *e.g.,* humans. In particular embodiments, pharmaceutical compositions comprise a gamma secretase modulators or a derivative thereof, formulated with one or more pharmaceutically-acceptable carriers, diluents, and/or excipients. It will also be understood that, if desired, the compositions of the invention may be administered in combination with other agents as well, such as, *e.g.,* nucleic acids, proteins, small molecules, or pharmaceutically-active agents, adjunct therapies, *etc.* so long as the desired therapeutic effect is achieved. There is virtually no limit to other reagents that may also be included in the compositions, provided that the additional reagents do not adversely affect the desired cancer therapy.

In particular embodiments, compositions comprise pharmaceutically acceptable formulations with therapeutically effective amounts of gamma secretase modulators or derivatives thereof; or prodrugs, solvates, stereoisomers, racemates, or tautomers of gamma secretase modulators or derivatives thereof, formulated with one or more pharmaceutically acceptable carriers (additives), other active agents, and/or diluents.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. As used herein "pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, surfactant, or emulsifier which has been approved by the United States Food and Drug Administration as being acceptable for use in humans or domestic animals. Exemplary pharmaceutically acceptable carriers include, but are not limited to, to sugars, such as lactose, glucose and sucrose; starches, such as corn starch and potato starch; cellulose, and its derivatives, such as sodium carboxymethyl cellulose, ethyl cellulose and cellulose acetate; tragacanth; malt; gelatin; talc; cocoa butter, waxes, animal and vegetable fats, paraffins, silicones, bentonites, silicic acid, zinc oxide; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and any other compatible substances employed in pharmaceutical formulations.

In particular embodiments, compounds contemplated herein exist in free base or acid form and can be converted to their pharmaceutically acceptable salts by treatment with the appropriate inorganic or organic base or acid by methods known to one skilled in the art. "Pharmaceutically acceptable salt" includes both acid and base addition salts. "Pharmaceutically acceptable acid addition salt" refers to those salts which retain the biological effectiveness and properties of the free bases, which are not biologically or otherwise undesirable, and which are formed with inorganic acids such as, but are not limited to, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, and the like.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2 dimethylaminoethanol, 2 diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, N ethylpiperidine, polyamine resins and the like. Particularly preferred organic bases are isopropylamine, diethylamine, ethanolamine, trimethylamine, dicyclohexylamine, choline and caffeine.

Salts of the compounds of the invention can be converted to their free base or acid form by standard techniques.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

In particular embodiments, a pharmaceutical composition contemplated herein is formulated so as to allow the active ingredients contained therein to be bioavailable upon administration of the composition to a subject. In one embodiment, pharmaceutical compositions can be prepared by combining a gamma secretase modulator or derivative thereof, with an appropriate pharmaceutically acceptable carrier, diluent or excipient, and may be formulated into preparations in solid, semi solid, liquid, gels, and microspheres. However, in certain embodiments the subject compounds may be simply dissolved or suspended in sterile water of physiological saline, Ringer's solution, or 0.9% NaCl.

Solid formulations of the compositions contemplated herein, include dragees, capsules, pills and granules, optionally scored or prepared with coatings and shells, such as enteric coatings and other coatings. Solid dosage forms may also be formulated so as to provide slow or controlled release of the compound. Thus, solid formulations could include any material that could provide a desired release profile of the compound, including but not limited to hydroxypropylmethyl cellulose in varying proportions, or other polymer matrices, liposomes and/or microspheres.

Coated, gel, or encapsulating formulations of gamma secretase modulators or derivatives thereof may also be formulated to deliver pulsatile, sustained, or extended release. For example, one method of pulsatile release could be achieved by layering multiple coatings of gamma secretase modulators or derivatives thereof, or by incorporating gamma secretase modulators or derivatives thereof within different regions of the formulation having different release times.

Liquid dosage formulations contemplated herein include pharmaceutically acceptable emulsions, microemulsions, solutions, suspensions, syrups and elixirs. In addition, the liquid dosage formulations may contain inert diluents commonly used in the art, including but not limited to water or other solvents; solubilizing agents and emulsifiers, such as ethyl alcohol, isopropyl alcohol, ethyl carbonate, ethyl acetate, benzyl alcohol, benzyl benzoate, propylene glycol, 1,3-butylene glycol; oils (in particular, cottonseed, groundnut, corn, germ, olive, castor and sesame oils); glycerol; tetrahydrofuryl alcohol; polyethylene glycols; and fatty acid esters of sorbitan, and mixtures thereof.

Suspensions formulations include, without limitation, ethoxylated isostearyl alcohols; polyoxyethylene sorbitol and sorbitan esters; microcrystalline cellulose; aluminum metahydroxide; bentonite; agar-agar; tragacanth; and mixtures thereof.

Injectable depot formulations can be made by forming microencapsulated matrices of the composition in biodegradable polymers. Examples of biodegradable polymers include, but are not limited to polylactide-polyglycolide, poly(orthoesters) and poly(anhydrides). The ratio of composition to polymer and the nature of the particular polymer employed can affect the rate of release of gamma secretase modulators or derivatives thereof from the composition. Depot injectable formulations can also be prepared by entrapping the drug in liposomes or microemulsions.

Proper fluidity of liquid, suspension and other formulations of the compounds can be maintained by the use of coating materials such as lecithin; by the maintenance of the required particle size in the case of dispersions; or by the use of surfactants.

Formulations may also include anti-contamination agents for the prevention of microorganism contamination. Anti-contamination agents may include but are not limited to antibacterial and antifungal agents, for example, paraben, chlorobutanol, phenol sorbic acid, antibiotics, and the like.

Formulations may also be sterilized by, for example, by filtration through a bacteria-retaining filter, or by incorporating sterilizing agents in the form of sterile solid formulations which can be dissolved in sterile water, or some other sterile medium immediately before use or formulation.

Formulations may also be endotoxin free. As used herein, the term "endotoxin free" refers to compositions or formulations that contain at most trace amounts (i.e., amounts having no adverse physiological effects to a subject) of endotoxin, and preferably undetectable amounts of endotoxin. By "substantially free of endotoxin" is meant that there is less endotoxin per dose of cells than is allowed by the FDA for a biologic, which is a total endotoxin of 5 EU/kg body weight per day, which for an average 70 kg person is 350 EU per total dose of cells. In one embodiment, the term "endotoxin free" refers to a composition or formulation that is at least 95%, at least 96%, at least 97%, at least 98%, at least 99%, or 100% endotoxin free. Endotoxins are toxins associated with certain bacteria, typically gram-negative bacteria, although endotoxins may be found in gram-positive bacteria, such as *Listeria monocytogenes.* The most prevalent endotoxins are lipopolysaccharides (LPS) or lipooligosaccharides (LOS) found in the outer membrane of various Gram-negative bacteria, and which represent a central pathogenic feature in the ability of these bacteria to cause disease. Small amounts of endotoxin in humans can produce fever, a lowering of the blood pressure, and activation of inflammation and coagulation, among other adverse physiological effects. Therefore, it is often desirable to remove most or all traces of endotoxin from drug product containers, because even small amounts may cause adverse effects in humans.

Pharmaceutical compositions may further comprise one or more components that enhance the bioavailability of the active ingredients of the composition, e.g., penetration enhancers, stabilizing agents, and one or more components that provide slow or controlled release of the gamma secretase modulator or derivative thereof in a composition, e.g., biocompatible polymers and/or gels.

In particular embodiments, compositions comprising penetration enhancers will facilitate the delivery of the composition across biological barriers. A "penetration enhancer" or "permeability enhancer" includes a polyol such as polyethylene glycol (PEG), glycerol (glycerin), maltitol, sorbitol *etc.;* diethylene glycol monoethyl ether, azone, benzalkonium chloride (ADBAC), cetylperidium chloride, cetylmethylammonium bromide, dextran sulfate, lauric acid, menthol, methoxysalicylate, oleic acid, phosphatidylcholine, polyoxyethylene, polysorbate 80, sodium glycholate, sodium lauryl sulfate, sodium salicylate, sodium taurocholate, sodium taurodeoxycholate, sulfoxides, sodium deoxycholate, sodium glycodeoxycholate, sodium taurocholate and surfactants such as sodium lauryl sulfate, laureth-9, cetylpyridinium chloride and polyoxyethylene monoalkyl ethers, benzoic acids, such as sodium salicylate and methoxy salicylate, fatty acids, such as lauric acid, oleic acid, undecanoic acid and methyl oleate, fatty alcohols, such as octanol and nonanol, laurocapram, cyclodextrins, thymol, limonene, urea, chitosan and other natural and synthetic polymers.

Suitable polyols for inclusion in the solutions include glycerol and sugar alcohols such as sorbitol, mannitol or xylitol, polyethylene glycol and derivatives thereof. In some embodiments the composition further includes a preservative. Accepted preservatives such as benzalkonium chloride and disodium edetate (EDTA) are included in the compositions of the invention in concentrations sufficient for effective antimicrobial action, about 0.0001 to 0.1%, based on the weight of the composition.

In particular embodiments, compositions comprise stabilizers to increase the therapeutic lifetime of the compositions *in vivo.* Exemplary stabilizers include fatty acids, fatty alcohols, alcohols, long chain fatty acid esters, long chain ethers, hydrophilic derivatives of fatty acids, polyvinyl pyrrolidones, polyvinyl ethers, polyvinyl alcohols, hydrocarbons, hydrophobic polymers, moisture-absorbing polymers, and combinations thereof. In further embodiments, the chosen stabilizer changes the hydrophobicity of the formulation (*e.g.*, oleic acid, waxes), or improves the mixing of various components in the formulation (*e.g.,* ethanol), affects the moisture level in the formula (*e.g.,* PVP or polyvinyl pyrrolidone), affects the mobility of the phase (substances with melting points higher than room temperature such as long chain fatty acids, alcohols, esters, ethers, amides etc. or mixtures thereof; waxes), and/or improves the compatibility of the formula with encapsulating materials (*e.g.*, oleic acid or wax). In other embodiments, stabilizers are present in sufficient amounts to inhibit the degradation of the gamma secretase modulators or derivatives thereof in a composition. Examples of such stabilizing agents, include, but are not limited to: (a) about 0.5% to about 2% w/v glycerol, (b) about 0.1% to about 1% w/v methionine, (c) about 0.1% to about 2% w/v monothioglycerol, (d) about 1 mM to about 10 mM EDTA, (e) about 0.01% to about 2% w/v ascorbic acid, (f) 0.003% to about 0.02% w/v polysorbate 80, (g) 0.001% to about 0.05% w/v. polysorbate 20, (h) arginine, (i) heparin, (j) dextran sulfate, (k) cyclodextrins, (1) pentosan polysulfate and other heparinoids, (m) divalent cations such as magnesium and zinc; or (n) combinations thereof.

In particular embodiments, compositions are formulated as controlled release formulations. In general, controlled release drug formulations impart control over the release of drug with respect to site of release and time of release *in vivo.* Controlled release includes to immediate release, delayed release, sustained release, extended release, variable release, pulsatile release and bi-modal release. Advantages offered by controlled release include: less frequent dosing; more efficient drug utilization; localized drug delivery by placement of a delivery device or formulation at a treatment site *in vivo;* and the opportunity to administer and release two or more different drugs, each having a unique release profile, or to release the same drug at different rates or for different durations, by means of a single dosage unit.

Controlled release formulations may be made by formulating the compositions with biocompatible polymers, viscosity agents, gels, paints, foams, xerogels, microparticles, hydrogels, nanocapsules, and thermoreversible gels, or combinations thereof. In particular embodiments, the polymer or gels are biodegradable. Release properties are often controlled by the particular combination of polymers or gels used to formulate the composition. These methods are well known in the art.

Exemplary polymers suitable for formulating the inventive compositions include, but are not limited to polyamides, polycarbonates, polyalkylenes (polyethylene glycol (PEG)), polymers of acrylic and methacrylic esters, polyvinyl polymers, polyglycolides, polysiloxanes, polyurethanes and co-polymers thereof, celluloses, polypropylene, polyethylenes, polystyrene, polymers of lactic acid and glycolic acid, polyanhydrides, poly(ortho)esters, poly(butic acid), poly(valeric acid), poly(lactide-co-caprolactone), polysaccharides, proteins, polyhyaluronic acids, polycyanoacrylates, and blends, mixtures, or copolymers thereof.

In particular embodiments, the polymer is an ABA-type or BAB-type triblock copolymers or mixtures thereof, wherein the A-blocks are relatively hydrophobic and comprise biodegradable polyesters or poly(orthoester), and the B-blocks are relatively hydrophilic and comprise polyethylene glycol (PEG). The biodegradable, hydrophobic A polymer block comprises a polyester or poly(ortho ester), in which the polyester is synthesized from monomers selected from the group consisting of D,L-lactide, D-lactide, L-lactide, D,L-lactic acid, D-lactic acid, L-lactic acid, glycolide, glycolic acid, ε-caprolactone, ε-hydroxyhexanoic acid, γ-butyrolactone, γ-hydroxybutyric acid, δ-valerolactone, δ-hydroxyvaleric acid, hydroxybutyric acids, malic acid, and copolymers thereof.

Exemplary viscosity agents suitable for use in formulating compositions include, but are not limited to, hydroxypropyl methylcellulose, hydroxyethyl cellulose, polyvinylpyrrolidone, carboxymethyl cellulose, polyvinyl alcohol, sodium chondroitin sulfate, sodium hyaluronate, acacia (gum arabic), agar, aluminum magnesium silicate, sodium alginate, sodium stearate, bladderwrack, bentonite, carbomer, carrageenan, Carbopol, xanthan, cellulose, microcrystalline cellulose (MCC), ceratonia, chitin, carboxymethylated chitosan, chondrus, dextrose, furcellaran, gelatin, Ghatti gum, guar gum, hectorite, lactose, sucrose, maltodextrin, mannitol, sorbitol, honey, maize starch, wheat starch, rice starch, potato starch, gelatin, sterculia gum, xanthum gum, gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethyl-cellulose (HPMC), sodium carboxymethyl-cellulose (CMC), silicon dioxide, or polyvinylpyrrolidone (PVP: povidone).

Suitable gelling agents for use in preparation of the gel formulation include, but are not limited to, celluloses, cellulose derivatives, cellulose ethers (*e.g.*, carboxymethylcellulose, ethylcellulose, hydroxyethylcellulose, hydroxymethylcellulose, hydroxypropylmethylcellulose, hydroxypropylcellulose, methylcellulose), guar gum, xanthan gum, locust bean gum, alginates (*e.g.*, alginic acid), silicates, starch, tragacanth, carboxyvinyl polymers, carrageenan, paraffin, petrolatum, glycerin-based gels, glycerin-derived compounds, conjugated, or crosslinked gels, matrices, hydrogels, and polymers, as well as gelatins and their derivatives, and various native and synthetic hydrogel and hydrogel-derived compounds, and any combinations or mixtures thereof.

In a particular embodiment, compositions contemplated herein comprise an effective amount of one or more gamma secretase modulators or derivatives thereof, alone or in combination with one or more other therapeutic agents or modalities. Thus, the compositions may be administered individually or in combination with each other and/or with other known cancer treatments, such as radiation therapy, chemotherapy, transplantation, immunotherapy, hormone therapy, photodynamic therapy, *etc.* The compositions may also be administered in combination with antibiotics. Such therapeutic agents may be accepted in the art as a standard treatment for a particular disease state as described herein, such as a particular cancer. Exemplary therapeutic agents contemplated include cytokines, growth factors, NSAIDs, DMARDs, anti-inflammatories, chemotherapeutics, radiotherapeutics, therapeutic antibodies, or other active and ancillary agents.

In certain embodiments, compositions contemplated herein may be administered in conjunction with any number of chemotherapeutic agents. Illustrative examples of chemotherapeutic agents include alkylating agents such as thiotepa and cyclophosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine resume; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin and its pegylated formulations, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel (TAXOL®, Bristol-Myers Squibb Oncology, Princeton, N.J.) and doxetaxel (TAXOTERE®., Rhne-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; aminopterin; xeloda; ibandronate; CPT-11; topoisomerase inhibitor RFS 2000; difluoromethylomithine (DMFO); retinoic acid derivatives such as Targretin™ (bexarotene), Panretin™ (alitretinoin) ; ONTAK™ (denileukin diftitox) ; esperamicins; capecitabine; and pharmaceutically acceptable salts, acids or derivatives of any of the above. Also included in this definition are anti-hormonal agents that act to regulate or inhibit hormone action on cancers such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

A variety of other therapeutic agents may be used in conjunction with the compositions contemplated herein. In one embodiment, the compositions contemplated herein are administered with nonsteroidal anti-inflammatory drugs (NSAIDS) including aspirin, ibuprofen, naproxen, methotrexate, sulfasalazine, leflunomide, anti-TNF medications, cyclophosphamide, and mycophenolate.

Other exemplary NSAIDs are chosen from the group consisting of ibuprofen, naproxen, naproxen sodium, COX-2 inhibitors such as VIOXX® (rofecoxib) and CELEBREX® (celecoxib), and sialylates. Exemplary analgesics are chosen from the group consisting of acetaminophen, oxycodone, tramadol of proporxyphene hydrochloride. Exemplary glucocorticoids are chosen from the group consisting of cortisone, dexamethasone, hydrocortisone, methylprednisolone, prednisolone, or prednisone. Exemplary biological response modifiers include molecules directed against cell surface markers, cytokine inhibitors, such as the TNF antagonists, adalimumab (HUMIRA®) and infliximab (REMICADE®), chemokine inhibitors and adhesion molecule inhibitors. The biological response modifiers include monoclonal antibodies as well as recombinant forms of molecules. Exemplary DMARDs include azathioprine, cyclophosphamide, cyclosporine, methotrexate, penicillamine, leflunomide, sulfasalazine, hydroxychloroquine, Gold (oral (auranofin) and intramuscular) and minocycline. Illustrative examples of therapeutic antibodies suitable for combination with compositions contemplated herein, include but are not limited to, bavituximab, bevacizumab (avastin), bivatuzumab, blinatumomab, conatumumab, daratumumab, duligotumab, dacetuzumab, dalotuzumab, elotuzumab (HuLuc63), gemtuzumab, ibritumomab, indatuximab, inotuzumab, lorvotuzumab, lucatumumab, milatuzumab, moxetumomab, ocaratuzumab, ofatumumab, rituximab, siltuximab, teprotumumab, and ublituximab.

In particular embodiments, the compositions contemplated herein are administered with proteasome inhibitors. The term "proteasome inhibitor" refers to any substance which directly or indirectly inhibits the 20S and/or 26S proteasome or an activity thereof. In particular embodiments, proteasome inhibition is specific, *i.e.,* the proteasome inhibitor inhibits proteasome activity at a concentration that is lower than the concentration of the inhibitor required to produce another, unrelated biological effect. Illustrative examples of proteasome inhibitors that can administered with the compositions described herein include, but are not limited to, bortezomib (Velcade, PS-341), carfilzomib (Kyprolis), oprozomib (ONX 0912), delanzomib (CEP-18770), ixazomib citrate (MLN9708), marizomib (NPI-0052; salinosporamide A), dihydroeponemycin, epoxomicin, ONX-914 (PR-957), syringolin A, TMC-95A, argryin A, disulfiram, epigallocatechin-3-gallate, MG-132, lactacystin, HBX41108, MG-262, MG-115, AM114, MLN2238, AM114, gliotoxin, P005091, PSI, omuralide, AdaAhx3L3VS, 8-hydroxyquinoline hemisulfate salt hemihydrate, and clasto-lactacystin β-lactone.

In certain embodiments, the compositions contemplated herein are administered with steroids, e.g. glucorticoids or glucorticoid receptor agonists. Illustrative examples of glucocorticoids and glucocorticoid receptor agonists suitable for use in the compositions and methods contemplated herein include, but are not limited to, medrysone, alclometasone, alclometasone dipropionate, amcinonide, beclometasone, beclomethasone dipropionate, betamethasone, betamethasone benzoate, betamethasone valerate, budesonide, ciclesonide, clobetasol, clobetasol butyrate, clobetasol propionate, clobetasone, clocortolone, cloprednol, cortisol, cortisone, cortivazol, deflazacort, desonide, desoximetasone, desoxycortone, desoxymethasone, dexamethasone, diflorasone, diflorasone diacetate, diflucortolone, diflucortolone valerate, difluorocortolone, difluprednate, fluclorolone, fluclorolone acetonide, fludroxycortide, flumetasone, flumethasone, flumethasone pivalate, flunisolide, flunisolide hemihydrate, fluocinolone, fluocinolone acetonide, fluocinonide, fluocortin, fluocoritin butyl, fluocortolone, fluorocortisone, fluorometholone, fluperolone, fluprednidene, fluprednidene acetate, fluprednisolone, fluticasone, fluticasone propionate, formocortal, halcinonide, halometasone, hydrocortisone, hydrocortisone acetate, hydrocortisone aceponate, hydrocortisone buteprate, hydrocortisone butyrate, loteprednol, meprednisone, 6a-methylprednisolone, methylprednisolone, methylprednisolone acetate, methylprednisolone aceponate, mometasone, mometasone furoate, mometasone furoate monohydrate, paramethasone, prednicarbate, prednisolone, prednisone, prednylidene, rimexolone, tixocortol, triamcinolone, triamcinolone acetonide and ulobetasol, as well as combinations thereof.

In particular embodiments, the compositions contemplated herein are administered with one or more immunomodulatory drugs (IMiDs). Exemplary IMiDs include thalidomide and derivatives thereof. The term "thalidomide " refers to drugs or pharmaceutical formulations comprising the active thalidomide compound 2-(2,6-dioxopiperidin-3-yl)-1H-isoindole-1,3(2H)-dione. Thalidomide derivatives thereof refer to structural variants of thalidomide that have a similar biological activity such as, for example, without limitation, lenalidomide (REVLIMID™) ACTIMID™ (Celgene Corporation), and POMALYST™ (Celgene Corporation), and the compounds disclosed in US5712291, WO02068414, and WO2008154252, each of which is incorporated herein by reference in its entirety. Illustrative examples of IMiDs that may be administered with the compositions contemplated herein include, but are not limited to, thalidomide, lenalidomide, pomalidomide, linomide, CC-1088, CDC-501, and CDC-801.

In certain embodiments, the compositions described herein are administered in conjunction with one or more cytokines. A "cytokine" refers to proteins released by one cell population that act on another cell as intercellular mediators. Illustrative examples of such cytokines are lymphokines, monokines, and traditional polypeptide hormones. Included among the cytokines are growth hormones such as human growth hormone, hepatic growth factor; tumor necrosis factor-alpha and -beta; mullerian-inhibiting substance; inhibin; activin; erythropoietin (EPO); osteoinductive factors; interferons such as interferon-alpha, beta, and-gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; IL-15, a tumor necrosis factor such as TNF-alpha or TNF-beta; and other polypeptide factors including LIF and kit ligand (KL). As used herein, the term cytokine includes proteins from natural sources or from recombinant cell culture, and biologically active equivalents of the native sequence cytokines.

In particular embodiments, the compositions contemplated herein comprise a concentration of one or more pharmaceutically active ingredients (*i.e.,* a gamma secretase modulator or inhibitor or derivative thereof; and optionally pharmaceutically acceptable salts, prodrugs, solvates, stereoisomers, racemates, or tautomers thereof) of between about 0.01% to about 90%, between about 0.01% to about 50%, between about 0.1% to about 70%, between about 0.1% to about 50%, between about 0.1% to about 40%, between about 0.1% to about 30%, between about 0.1% to about 20%, between about 0.1% to about 10%, or between about 0.1% to about 5%, of each active ingredient, by weight of the composition.

In certain embodiments, the compositions described herein have a concentration of each active pharmaceutical agent between about 1% to about 50%, between about 5% to about 50%, between about 10% to about 40%, or between about 10% to about 30%, of the active ingredient, or pharmaceutically acceptable salt, prodrug, solvate, stereoisomer, racemate, or tautomer thereof, by weight of the composition.

In some embodiments, the formulations have a concentration of active pharmaceutical ingredient of between about 0.1 to about 70 mg/mL, between about 0.5 mg/mL to about 70 mg/mL, between about 0.5 mg/mL to about 50 mg/mL, between about 0.5 mg/mL to about 20 mg/mL, between about 1 mg to about 70 mg/mL, between about 1 mg to about 50 mg/mL, between about 1 mg/mL and about 20 mg/mL, between about 1 mg/mL to about 10 mg/mL, or between about 1 mg/mL to about 5 mg/mL, of the active agent, or pharmaceutically acceptable salt, prodrug, solvate, stereoisomer, racemate, or tautomer thereof, by volume of the formulation.

In one embodiment, the formulations additionally provide an immediate release of one or more pharmaceutically active ingredients (*i.e.,* gamma secretase modulator or inhibitor or derivatives thereof, or pharmaceutically acceptable salts, prodrugs, solvates, stereoisomers, racemates, or tautomers thereof) from the composition, or within 1 minute, or within 5 minutes, or within 10 minutes, or within 15 minutes, or within 30 minutes, or within 60 minutes or within 90 minutes.

In another embodiment, a therapeutically effective amount of at least one pharmaceutically active ingredient is released from the composition immediately, or within 1 minute, or within 5 minutes, or within 10 minutes, or within 15 minutes, or within 30 minutes, or within 60 minutes or within 90 minutes.

In yet another embodiment, a composition is formulated as an extended release formulation. In certain embodiments, diffusion of at least one pharmaceutically active ingredient from the formulation occurs for a time period exceeding 5 minutes, 15 minutes, 30 minutes, 1 hour, 4 hours, 6 hours, 12 hours, 18 hours, 1 day, 2 days, 3 days, 4 days, 5 days, 6 days, 7 days, 10 days, 12 days, 14 days, 18 days, 21 days, 25 days, 30 days, 45 days, 2 months 3 months 4 months 5 months 6 months 9 months or 1 year.

In particular embodiments, a therapeutically effective amount of at least one pharmaceutically active ingredient is released from the formulation for a time period exceeding 5 minutes, 15 minutes, 30 minutes, 1 hour, 4 hours, 6 hours, 12 hours, 18 hours, 1 day, 2 days, 3 days, 4 days, 5 days, 6 days, 7 days, 10 days, 12 days, 14 days, 18 days, 21 days, 25 days, 30 days, 45 days, 2 months 3 months 4 months 5 months 6 months 9 months or 1 year.

In further embodiments, the formulation provides both an immediate release and an extended release formulation. In particular embodiments, the formulation contains a 0.25:1 ratio, a 0.5:1 ratio, a 1:1 ratio, a 1:2 ratio, a 1:3, a 1:4 ratio, a 1:5 ratio, a 1:7 ratio, a 1:10 ratio, a 1:15 ratio, or a 1:20 ratio of immediate release and extended release formulations. In a further embodiment the formulation provides an immediate release of a first pharmaceutically active ingredient and an extended release of a second pharmaceutically active ingredient or other therapeutic agent.

In additional embodiments, the formulation provides a 0.25:1 ratio, a 0.5:1 ratio, a 1:1 ratio, a 1:2 ratio, a 1:3, a 1:4 ratio, a 1:5 ratio, a 1:7 ratio, a 1:10 ratio, a 1:15 ratio, or a 1:20 ratio of immediate release and extended release formulations of one or more pharmaceutically active ingredients.

The combination of immediate release, delayed release and/or extended release compositions or formulations may be combined with other pharmaceutical agents, as well as the excipients, diluents, stabilizers, carrier agents and other components disclosed elsewhere herein. As such, depending upon the components of a composition, the thickness or viscosity desired, or the mode of delivery chosen, alternative aspects of the embodiments disclosed herein are combined with the immediate release, delayed release and/or extended release embodiments accordingly.

Additional methods of formulating compositions are known to the skilled artisan, for example, as described in the Physicians Desk Reference, 62nd edition. Oradell, NJ: Medical Economics Co., 2008; Goodman & Gilman's The Pharmacological Basis of Therapeutics, Eleventh Edition. McGraw-Hill, 2005; Remington: The Science and Practice of Pharmacy, 20th Edition. Baltimore, MD: Lippincott Williams & Wilkins, 2000; and The Merck Index, Fourteenth Edition. Whitehouse Station, NJ: Merck Research Laboratories, 2006; each of which is hereby incorporated by reference in relevant parts.

### E. Administration

In particular embodiments, a method of treating a subject with a B cell condition or disorder or preventing BCMA shedding from a B cell in a subject is contemplated comprising administering to the subject a gamma secretase modulator or a derivative thereof. Compositions contemplated herein may be administered as one or more solids, semi-solids, gels, or liquids, or combination thereof. For example, a gamma secretase modulator or derivative thereof and other pharmaceutically active agents may be individually formulated for intravenous administration in a liquid dosage form or for oral administration as a single tablet or capsule or as a combination of one or more tablets, capsules, or other dosage forms. The specific amount/dosage regimen will vary depending on the weight, gender, age and health of the individual; the formulation, the biochemical nature, bioactivity, bioavailability and the side effects of the agents and the number and identity of the agents in the complete therapeutic regimen.

As used herein, the terms "administering," "administer," or "administration" refer to deliver one or more compounds or compositions to a subject parenterally, enterally, or topically. Illustrative examples of parenteral administration include, but are not limited to, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion. Illustrative examples of enteral administration include, but are not limited to oral, inhalation, intranasal, sublingual, and rectal administration. Illustrative examples of topical administration include, but are not limited to, transdermal and vaginal administration.

In particular embodiments, an agent or composition is administered parenterally, optionally by intravenous administration or oral administration to a subject.

In various embodiments, the development of suitable dosing and treatment regimens for using the particular compositions contemplated herein in a variety of treatment regimens including, e.g., oral, parenteral, intravenous, intranasal, and intramuscular administration and formulation, is well known in the art. In certain embodiments, a gamma secretase modulator is administered intravenously to a subject. In particular embodiments, a gamma secretase modulator is administered intramuscularly to a subject. In some embodiments, a gamma secretase modulator is administered sublingually to a subject. In particular embodiments, a gamma secretase modulator is administered subcutaneously to a subject.

In particular embodiments, a gamma secretase modulator or derivative thereof is administered orally to a subject. The agent can be administered to the subject at a dose in the range of about 1-100 mg, about 1-50 mg, about 50-100 mg, about 1-5 mg, about 5-10 mg, about 10-15mg, about 15-20 mg, about 20-30 mg, about 30-40 mg, about 40-50 mg, about 50-60 mg, about 60-70 mg, about 70-80 mg, about 80-90 mg, or about 90-100 mg or more. In certain embodiments, the agent is administered in a dose of about 1 mg, about 2 mg, about 2.5 mg, about 3 mg, about 4 mg, about 5 mg, about 6 mg, about 7 mg, about 8 mg, about 9 mg, about 10 mg, about 15 mg, about 20 mg, about 25 mg, about 50 mg, or about 100 mg or more. In some embodiments of the invention, an oral dose of an agent is administered to the subject at least once in a treatment cycle, at least once in a 28 day treatment cycle, at least once a week, at least once every other day, at least once a day, or at least twice a day.

In particular embodiments, a gamma secretase modulator or a derivative thereof is administered intravenously. The agent can be administered intravenously at a dose of about 0-100 mg, about 1 -50 mg, about 50-100 mg, about 1-10 mg, about 10-20 mg, about 20-30 mg, about 30-40 mg, about 40-50 mg, about 50 - 60 mg, about 60-70 mg, about 70-80 mg, about 80-90 mg, or about 90-100 mg or more. In certain embodiments, the intravenous dose of agent is about one mg, about two mg, about three mg, about four mg, about five mg, about six mg, about seven mg, about eight mg, about nine mg, about ten mg, about 15 mg, about 20 mg, about 25 mg, about 30 mg, about 35 mg, about 40 mg, about 45 mg, about 50 mg, about 60 mg, about 70 mg, about 80 mg, about 90 mg, or about 100 mg or more. Doses of agents can be delivered intravenously in any pharmaceutically suitable vehicles for injection or infusion known in the art.

In some embodiments, the agent can be administered intravenously at a dose of about 0-100 mg/m², about 1 -50 mg/m², about 50-100 mg/m², about 1-10 mg/m², about 10-20 mg/m², about 20-30 mg/m², about 30-40 mg/m², about 40-50 mg/m², about 50 - 60 mg/m², about 60-70 mg/m², about 70-80 mg/m², about 80-90 mg/m², or about 90-100 mg/m² or more. In certain embodiments, the intravenous dose of agent is about one mg/m², about two mg/m², about three mg/m², about four mg/m², about five mg/m², about six mg/m², about seven mg/m², about eight mg/m², about nine mg/m², about ten mg/m², about 15 mg/m², about 20 mg/m², about 25 mg/m², about 30 mg/m², about 35 mg/m², about 40 mg/m², about 45 mg/m², about 50 mg/m², about 60 mg/m², about 70 mg/m², about 80 mg/m², about 90 mg/m², or about 100 mg/m² or more.

In some embodiments, the agent can be administered intravenously at a dose of about 0-10 mg/kg, about 0-5 mg/kg, about 5-10 mg/kg, about 0-1 mg/kg, about 1-2 mg/kg, about 2-3 mg/kg, about 3-4 mg/kg, about 4-5 mg/kg, about 5-6 mg/kg, about 6-7 mg/kg, about 7-8 mg/kg, about 8-9 mg/kg, or about 9-10 mg/kg or more. In certain embodiments, the intravenous dose of agent is about 0.05 mg/kg, about 0.1 mg/kg, about 0.15 mg/kg, about 0.2 mg/kg, about 0.25 mg/kg, about 0.3 mg/kg, about 0.35 mg/kg, about 0.4 mg/kg, about 0.45 mg/kg, about 0.5 mg/kg, about 0.55 mg/kg, about 0.6 mg/kg, about 0.65 mg/kg, about 0.7 mg/kg, about 0.75 mg/kg, about 0.8 mg/kg, about 0.85 mg/kg, about 0.9 mg/kg, about 0.95 mg/kg, about one mg/kg, about two mg/kg, about three mg/kg, about four mg/kg, about five mg/kg, about six mg/kg, about seven mg/kg, about eight mg/kg, about nine mg/kg, or about ten mg/kg or more.

In some embodiments, a gamma secretase modulator or a derivative thereof is administered at least once during a treatment cycle. In some embodiments, a gamma secretase modulator or a derivative thereof is administered to the subject on the same days. In some embodiments, a gamma secretase modulator or a derivative thereof is administered to the subject on the different days. In some embodiments, a gamma secretase modulator or a derivative thereof is administered to the subject on the same days and on different days according to treatment schedules.

In particular embodiments, an agent is administered to the subject over one or more treatment cycles. A treatment cycle can be at least two, at least three, at least four, at least five, at least six, at least seven, at least 14, at least 21, at least 28, at least 48, or at least 96 days or more. In one embodiment, a treatment cycle is 28 days. In certain embodiments, the agents are administered over the same treatment cycle or concurrently over different treatment cycles assigned for each agent. In various embodiments, the treatment cycle is determined by a health care professional based on conditions and needs of the subject.

In some embodiments, an agent is administered on at least one day, at least two days, at least three days, at least four days, at least five days, at least six days, at least seven days, at least eight days, at least nine days, at least ten days, at least eleven days, at least twelve days, at least 13 days, at least 14 days, at least 21 days, or all 28 days of a 28 day treatment cycle. In particular embodiments, an agent is administered to a subject once a day. In other particular embodiments, an agent is administered twice a day. In certain embodiments an agent is administered more than twice a day.

In particular embodiments, an agent is administered on day 1, day 2, day 8, day 9, day 15, and day 16 of a 28 day treatment cycle. In some embodiments, a gamma secretase modulator or a derivative thereof is administered on day 1, day 2, day 8, day 9, day 15, and day 16 of a 28 day treatment cycle.

The number of times a composition is administered to a subject in need thereof depends on the discretion of a medical professional, the disorder, the severity of the disorder, and the subject's response to the formulation. In some embodiments, a composition disclosed herein is administered once to a subject in need thereof with a mild acute condition. In some embodiments, a composition disclosed herein is administered more than once to a subject in need thereof with a moderate or severe acute condition. In the case wherein the subject's condition does not improve, upon the doctor's discretion the composition may be administered chronically, that is, for an extended period of time, including throughout the duration of the subject's life in order to ameliorate or otherwise control or limit the symptoms of the subject's disease or condition.

In the case wherein the subject's status does improve, upon the doctor's discretion the composition may administered continuously; or, the dose of drug being administered may be temporarily reduced or temporarily suspended for a certain length of time (*i.e.,* a "drug holiday"). The length of the drug holiday varies between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, and 365 days. The dose reduction during a drug holiday may be from 10%-100%, including by way of example only 10%, 15%, 20%, 25%, 30%, 35%, 40%, 45%, 50%, 55%, 60%, 65%, 70%, 75%, 80%, 85%, 90%, 95%, and 100%.

### F. Methods

In various embodiments, the present invention contemplates B cell therapies comprising administering one or more gamma secretase modulators to a subject.

In one embodiment, the methods contemplated herein comprise treating or preventing a B cell condition or disorder in a subject comprising administering to the subject one or more gamma secretase modulators or a derivative thereof.

In a still further aspect, the invention provides methods of treating or preventing or delaying a B-cell mediated condition disorder. The method includes administering to a subject in which such treatment or prevention or delay is desired, a composition of the invention in an amount sufficient to treat, prevent, or delay a tumorigenic or immunoregulatory condition in the subject. In some embodiments, the subject is a human. In other embodiments, the subject is a non-human mammal. In some embodiments, administration of the composition of the invention reduces or prevents gamma secretase-mediated cleavage of BCMA in the subject, which may result in one or more of cell death, inhibition, reduction, or cessation of cell proliferation.

Illustrative examples of B cell-related conditions or disorders suitable for treatment with the compositions or methods contemplated herein include, without limitation, autoimmune diseases involving inappropriate B cell activity and B cell lymphomas. B cell lymphomas include, without limitation, MM, plasmacytoma, WM, CLL, Hodgkins' lymphoma, follicular lymphomas, small non-cleaved cell lymphomas, endemic Burkitt's lymphoma, sporadic Burkitt's lymphoma, marginal zone lymphoma, extranodal mucosa-associated lymphoid tissue lymphoma, nodal monocytoid B cell lymphoma, splenic lymphoma, mantle cell lymphoma, large cell lymphoma, diffuse mixed cell lymphoma, immunoblastic lymphoma, primary mediastinal B cell lymphoma, pulmonary B cell angiocentric lymphoma, small lymphocytic lymphoma, lymphomatoid granulomatosis and post-transplant lymphoproliferative disorder, primary or immunocyte-associated amyloidosis, and monoclonal gammopathy of undetermined significance (MGUS).

Illustrative examples of B cell-related conditions or disorders suitable for treatment with the compositions or methods contemplated herein include, without limitation, disorders that are autoimmune in nature such as, for example, systemic lupus erythematosus, rheumatoid arthritis, myasthenia gravis, autoimmune hemolytic anemia, idiopathic thrombocytopenia purpura, anti-phospholipid syndrome, Chagas' disease, Grave's disease, Wegener's granulomatosis, poly-arteritis nodosa, Sjogren's syndrome, pemphigus vulgaris, scleroderma, multiple sclerosis, anti-phospholipid syndrome, ANCA associated vasculitis, Goodpasture's disease, Kawasaki disease, heavy-chain disease, and rapidly progressive glomerulonephritis.

Illustrative examples of hematological malignancies suitable for treatment with the compositions and methods contemplated herein include, but are not limited to MM, WM, leukemia, or lymphoma. Leukemias can include, but are not limited to, ALL, AML, CLL, CML, acute monocytic leukemia. Lymphomas can include, but are not limited to, Hodgkin's lymphomas, such as nodular sclerosis Hodgkin's lymphoma, mixed cellularity subtype Hodgkin's lymphoma, Lymphocyte rich Hodgkin's lymphoma, and lymphocyte depleted Hodgkin's Lymphoma; and non- Hodgkin's lymphoma, such as diffuse large B-cell lymphoma, primary mediastinal B-cell lymphoma, follicular lymphoma, CLL, mantle cell lymphoma, marginal zone B- cell lymphomas, Burkitt lymphoma, lymphoplasmacytic lymphoma, primary central nervous system lymphoma, T-cell lymphomas, and WM Plasma cell dyscrasias include, but are not limited to, multiple myeloma.

In various embodiments, methods of preventing or decreasing BCMA shedding from B cells is also contemplated, comprising administering to a subject, one or more gamma secretase modulators or a derivative thereof. Preventing or decreasing B cell shedding of BCMA with increase the amount of BCMA detected on B cells and reduce or decrease the amount of BCMA that is detectable in the serum. Without wishing to be bound by any particular theory, it is contemplated that reducing the shedding will enhance other therapies that target BCMA expressed on B cells, since if BCMA is shed, the B cell will no longer be efficiently targeted, but if the shedding is blocked or decreased more BCMA will be expressed on the surface of the B cells, thereby increasing the efficacy of the therapies that target BCMA expressed on B cells. Therapeutic agents that bind to BCMA and/or a cell expressing BCMA include, but are not limited to, therapeutic anti-BCMA antibodies or fragments thereof, anti-BCMA antibody-drug conjugates, and chimeric antigen receptor T cells (CAR-T cells) that binds to BCMA

In some embodiments, a gamma secretase modulator is administered to a subject undergoing a therapy that targets BCMA. In certain embodiments, the therapy that targets BCMA comprises administering a therapeutic agent that binds to BCMA. In particular embodiments, the agent binds to BCMA expressed by B cells. In some embodiments, the B cells are pathogenic B cells. In some embodiments, administration of the gamma secretase inhibitor increases the binding of the therapeutic agent to pathogenic B cells expressing BCMA by about 5%, about 10%, about 15%, about 20%, about 25%, about 30%, about 20%, about 25%, about 30%, about 35%, about 40%, about 45%, about 50%, about 55%, about 60%, about 65%, about 70%, about 75%, about 80%, about 85%, about 90%, about 95%, about 100%, about 1.5-fold, about 2-fold, about 2.5-fold, about 3-fold, about 4-fold, about 5-fold, about 10-fold, about 20-fold, about 30-fold, about 40-fold, about 50-fold, about 100-fold, or greater than 100-fold (including all ranges and values in-between) as compared to the binding of the therapeutic agent to pathogenic B cells in the absence of the gamma secretase inhibitor.

In particular embodiments, a gamma secretase modulator is administered to a subject undergoing a therapy that targets BCMA. In some embodiments, the therapy that targets BCMA comprises administering an agent that reduces the number of pathogenic B cells since they express BCMA. In some embodiments, administration of the gamma secretase inhibitor further reduces the amount of pathogenic B cells since they express BCMA in the subject undergoing the therapy that targets BCMA by about 5%, about 10%, about 15%, about 20%, about 25%, about 30%, about 20%, about 25%, about 30%, about 35%, about 40%, about 45%, about 50%, about 55%, about 60%, about 65%, about 70%, about 75%, about 80%, about 85%, about 90%, about 95%, or by about 100%, (including all ranges and values in-between) as compared to the reduction of pathogenic B cells achieved by the therapy that targets BCMA in the absence of the gamma secretase inhibitor.

In various embodiments, the methods contemplated herein comprise increasing the efficacy of a therapy in a subject being treated for a B cell condition or disorder comprising: administering the subject one or more gamma secretase modulators or a derivative thereof in addition to the existing treatment being provided to the subject. Therapies for a B cell condition or disorders include, but are not limited to, radiation therapy, chemotherapy, transplantation, immunotherapy, proteasome inhibitors, immunomodulatory agents, hormone therapy, or photodynamic therapy.

In certain embodiments, one or more gamma secretase modulators are administered to a subject to increase the efficacy of a therapy or treatment for a B cell condition or disorder that is autoimmune in nature. Therapies for B cell conditions that are autoimmune in nature include, but are not limited to, corticosteroids (e.g. prednisone, prednisolone and methylprednisolone), disease-modifying antirheumatic drugs (DMARDs; *e.g.,* methotrexate, hydroxycholorquine, sulfasalazine, leflunomide, cyclophosphamide and azathioprine), JAK inhibitors (*e.g.,* tofacitinib and ruxolitinib), and biologics (*e.g.,* tocilzumab, cerolizumab, etanercept, adalimumab, anakinra, abatacept, infliximab, rituximab), nonsteroidal anti-inflammatory drugs (NSAIDS; *e.g.* aspirin, ibuprofen, and naproxen), acetylcholinesterase inhibitors (e.g.,physostigmine, neostigmine, pyridostigmine, ambenonium, demecarium, rivastigmine, phenanthrene, galantamine, donepezil, tacrine, and edrophonium), cytostatics (e.g., folic acid analogs such as methotrexate, purine analogs such as azathioprine and mercaptopurine, and pyrimidine analogs such as fluorouracil), drugs that act on immunophilins (e.g. ciclosporin, tacrolimius, and sirolimius), and interferons such as IFN-beta.

In various embodiments, one or more gamma secretase modulators are administered to a subject to increase the efficacy of a therapy or a treatment for a B cell condition or disorder that is a hematological malignancy. Therapies for hematological malignancies include, but are not limited to, radiation therapy and chemotherapy (*e.g.* combination chemotherapy such as MOPP (combination of Mustargen, Oncovin (also known as vincristine), prednisone and procarbazine (also known as Matulane)), ABVD (combination of adriamycin, bleomycin, vinblastine, and dacarbazine), and CHOP (cyclophosphamide, doxorubicin, vincristine, prednisolone); treatment with alkylating agents such as, melphalan, cyclophosphamide, nitrosoureas, tetrazines, aziridines, cisplatins and derivatives, and non-classical alkylating agents such as bendamustine; cytostatics (e.g., folic acid analogs such as methotrexate, purine analogs such as azathioprine and mercaptopurine, and pyrimidine analogs such as fluorouracil); treatment with anti-microtuble agents such as vincristine and vinblastine, taxanes, pacilitaxel, docetaxel, and ligan; treatment with topoisomerase inhibitors such as irinotecan, topotecan, camptothecin, etoposide, doxorubicin, mitoxantrone, and teniposide, proteasome inhibitors such as bortezomib, carfilzomib and ixazomib, and immunomodulatory agents such as thalidomide, lenalidomide and pomalidomide.

Certain embodiments contemplate that an increase in the efficacy of a therapy can be readily determined and/or identified by one skilled in the art. In some embodiments, an improvement of the efficacy of a therapy for a B cell condition or disorder is an improvement, an alleviation, an amelioration, and/or a reduction of at least one sign or symptom of the B cell condition or disorder being treated, as compared to the therapy without the one or more gamma secretase modulators. In some embodiments, a subject who is receiving a therapy for a B cell condition or disorder is administered a gamma secretase inhibitor, and at least one sign or symptom of the B cell condition or disorder is further reduced by the therapy, as compared to treatment with the therapy alone. Symptoms of a B cell condition or disorder include, but are not limited to, generalized weakness and fatigue, anemia, dizziness, frequent or unexplained fever and infection, weight loss or loss of appetite, excessive and unexplained bruising, breathlessness, enlarged lymph nodes, liver, or spleen, pitting edema, joint inflammation, blood clots, skin rash, jaundice, itchy skin, joint pain, insomnia, heat sensitivity, muscle weakness, tremors, paralysis, difficulty speaking, difficulty breathing.

In particular embodiments, improved efficacy of a therapy includes an alleviation, abatement, amelioration, and/or reduction on at least one unwanted side effect of the therapy. Thus, in certain embodiments, one or more gamma secretase modulators are administered to a subject receiving a therapy for a B cell condition or disorder to abate, alleviate, ameliorate, and/or reduce at least one unwanted side effect of the therapy. Examples of unwanted side effects will be readily identified by those of skill in the art, and include, but are not limited to, increased risk or incidence of infection, increased risk or incidence of fever, immunosuppression, reduced immune function, and reduced antibody production.

In some embodiments, a gamma secretase inhibitor is administered to a subject receiving a therapy for a B cell condition or disorder, and tumor number and/or tumor volume is reduced as compared to treatment with the therapy alone. In particular embodiments, a gamma secretase modulator is administered to a subject receiving a therapy for a B cell condition or disorder, and the number of cancer cells in the subject is reduced as compared to treatment with the therapy alone. In various embodiments, a gamma secretase modulator is administered to a subject receiving a therapy for a B cell condition or disorder, the probability of remission of the B cell condition or disorder is increased with the addition of the gamma secretase modulator as compared to treatment with the therapy alone. In certain embodiments, a gamma secretase modulator is administered to a subject receiving a therapy for a B cell condition or disorder, and the probability of survival is increased as compared to the probability of survival from the therapy alone.

### EXAMPLES

### Example 1:Treatment of LAGκ-1A tumor cells With Gamma Secretase Inhibitor Causes a Reduction of Supernatant BCMA Levels

Cells were obtained from a human multiple myeloma LAGκ-1A tumor that was removed from the hind limb of a severe combined immunodeficient (SCID) mouse. Single cells were dissociated from the tumor, suspended, and cultured in 24-well or 96-well cell culture plates. For determination of BCMA concentrations, LAGκ-1A tumor cells were cultured at cell numbers of either 5x10⁵ cells or 2x10⁶ cells per well in a 24-well plate.

After two hours, the gamma secretase inhibitor E424354 from Lilly Pharmaceuticals (Indianapolis, Indiana) was added to the cell media. Cells were incubated without or with E424354 at concentrations between 256 pM and 20 µM. Specifically, concentrations of 256 pM, 1.28 nM, 6.4 nM, 32 nM, 160 nM, 800 nM, 4 µM, and 20 µM of E424354 were tested. Cells were incubated with these concentrations of E424354 for 48 hours, 72 hours, or 5 days. After the incubation, the cells were then centrifuged, and the supernatant was collected. Supernatant samples were analyzed by BCMA enzyme-linked immunosorbent assay (ELISA) obtained from R&D Systems, Minneapolis, MN, USA (catalogue #DY193E). Serum samples were diluted 1:50 and the BCMA ELISA assay was carried out according to the manufacturer's protocol. The ELISA plates were analyzed using a µQuant (Biotek Industries, Winooski, VT, USA) plate reader set to 450 nm with KC Junior software. Values represent the mean of triplicate samples on each specimen. This BCMA ELISA kit does not cross react with recombinant human APRIL or BAFF, recombinant human TACI/Fc or recombinant mouse BCMA/Fc or mouse BCMA

Supernatant BCMA levels were reduced in samples collected from cultured LAGκ-1A tumor cells treated with the gamma secretase inhibitor compared to samples from LAGκ-1A tumor cells treated with a vehicle control. When LAGκ-1A tumor cells cultured at a density of 5x10⁵ cells per well were incubated with the gamma secretase inhibitor for 48 hours (FIG. 2) or 72 hours (FIG. 3), or when LAGκ-1A tumor cells cultured at a density of 2x10⁶ cells per well were incubated with the gamma secretase inhibitor for 48 hours (FIG. 4), 72 hours (FIG. 5), or 5 days (FIG. 6), lower levels of supernatant BCMA were observed as compared to the control. Across all conditions tested (i.e., cell density, E424354 concentration, and incubation time), lower levels of supernatant BCMA were detected in samples collected from E424354 -treated cells as compared to cells without E424354 (controls). The effects of gamma secretase inhibitor were apparent even at very low (picomolar) concentrations of the gamma secretase inhibitor.

MTS assays were performed to assess effects of E424354 on cell growth and cell viability. LAGκ-1A tumor cells were cultured at 5x10⁴ cells per well in a 96-well plate. Cells were cultured for two hours at 37°C with 5% CO₂ before E424354 was added. Cells were incubated in E424354 for 24 hours (FIG. 7), 48 hours (FIG. 8), 72 hours (FIG. 9), or 5 days (FIG. 10). At all concentrations and lengths of drug exposure tested, LAGκ-1A tumor cells treated with the gamma secretase inhibitor showed no cytotoxic effects as assessed using an MTS assay cell proliferation assay, indicating that the reduced levels of BCMA in supernatant fluid from cells treated with the gamma secretase inhibitor E424354 were not due to cell death. Taken together, these data provide support for the ability of gramma secretase inhibitor to prevent shedding of BCMA off of myeloma tumor cells.

Although the foregoing invention has been described in some detail by way of illustration and example for purposes of clarity of understanding, it will be readily apparent to one of ordinary skill in the art in light of the teachings of this invention that certain changes and modifications may be made thereto without departing from the spirit or scope of the invention contemplated herein.

In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

### ITEMS

The invention is also characterized by the following items:
1. A method of treating or preventing an immune system dysfunction in a subject comprising administering to the subject one or more gamma secretase modulators or a derivative thereof.
2. A method of decreasing BCMA shedding from a plasma cell comprising administering one or more gamma secretase modulators or a derivative thereof to a subject having an immune system dysfunction, optionally wherein the immune system dysfunction is due to the expression of BCMA.
3. A method of increasing the efficacy of a therapy in a subject being treated for a B cell condition or disorder comprising: administering the subject one or more gamma secretase modulators or a derivative thereof in addition to the existing treatment being provided to the subject.
4. The method of item 1 or item 2, wherein the immune system dysfunction is a B cell condition or disorder.
5. The method of any one of items 2-4, wherein the B cell condition or disorder is selected from the group consisting of: MM, WM, CLL, B cell non-Hodgkin's lymphoma, plasmacytoma, Hodgkins' lymphoma, follicular lymphomas, small non-cleaved cell lymphomas, endemic Burkitt's lymphoma, sporadic Burkitt's lymphoma, marginal zone lymphoma, extranodal mucosa-associated lymphoid tissue lymphoma, nodal monocytoid B cell lymphoma, splenic lymphoma, mantle cell lymphoma, large cell lymphoma, diffuse mixed cell lymphoma, immunoblastic lymphoma, primary mediastinal B cell lymphoma, pulmonary B cell angiocentric lymphoma, small lymphocytic lymphoma, B cell proliferations of uncertain malignant potential, lymphomatoid granulomatosis, post-transplant lymphoproliferative disorder, an immunoregulatory disorder, rheumatoid arthritis, myasthenia gravis, idiopathic thrombocytopenia purpura, anti-phospholipid syndrome, Chagas' disease, Grave's disease, Wegener's granulomatosis, poly-arteritis nodosa, Sjogren's syndrome, pemphigus vulgaris, scleroderma, multiple sclerosis, anti-phospholipid syndrome, ANCA associated vasculitis, Goodpasture's disease, Kawasaki disease, autoimmune hemolytic anemia, and rapidly progressive glomerulonephritis, heavy-chain disease, primary or immunocyte-associated amyloidosis, or monoclonal gammopathy of undetermined significance.
6. The method of any one of items 2-4, wherein the B cell condition or disorder is a B cell malignancy.
7. The method of any one of items 2-4, wherein the B cell condition or disorder is a plasma cell malignancy.
8. The method of any one of items 2-4, wherein the B cell condition or disorder is selected from the group consisting of: MM, WM, CLL, and B-cell non-Hodgkin's lymphoma.
9. The method of any one of items 2-4, wherein the B cell condition or disorder is multiple myeloma.
10. The method of any one of items 2-4, wherein the B cell condition or disorder is an autoimmune disease.
11. The method of any one of items 2-4, wherein the B cell condition or disorder is systemic lupus erythematosus.
12. The method of any one of items 2-4, wherein the B cell condition or disorder is rheumatoid arthritis.
13. The method of any one of items 2-4, wherein the B cell condition or disorder is selected from the group consisting of: idiopathic thrombocytopenia purpura, myasthenia gravis, and autoimmune hemolytic anemia.
14. The method of any one of the preceding items, wherein the gamma secretase modulator is selected from the group consisting of: secretase inhibitor I (GSI I) Z-Leu-Leu-Norleucine; γ-secretase inhibitor II (GSI II); γ- secretase inhibitor III (GSI III), N-Benzyloxycarbonyl-Leu- leucinal, N-(2-Naphthoyl)-Val- phenylalaninal; γ-secretase inhibitor III (GSI IV); γ-secretase inhibitor III (GSI V), N- Benzyloxycarbonyl-Leu- phenylalaninal; γ-secretase inhibitor III (GSI VI), 1-(S)-endo-N-(1,3,3)-Trimethylbicyclo[2.2.1]hept-2-yl)-4-fluorophenyl Sulfonamide; γ-secretase inhibitor III (GSI VII), Menthyloxycarbonyl-LL-CHO; γ-secretase inhibitor III (GSI IX), (DAPT), N- [N-(3,5- Difluorophenacetyl-L- alanyl)]-S-phenylglycine t- Butyl Ester; γ-secretase inhibitor X (GSI X), {1S-Benzyl-4R-[1-(1S-carbamoyl-2-phenethylcarbamoyl)-1S-3-methylbutylcarb-amoyl]-2R-hydroxy-5-phenylpentyl}carbamic Acid tert-butyl Ester; γ- secretase inhibitor XI (GSI XI), 7-Amino-4-chloro-3-methoxyisocoumarin; γ-secretase inhibitor XII (GSI XII), Z-Ile-Leu-CHO; γ-secretase inhibitor XIII (GSI XIII), Z-Tyr-Ile-Leu- CHO; γ-secretase inhibitor XIV (GSI XIV), Z-Cys(t-Bu)-Ile-Leu-CHO; γ-secretase inhibitor XVI (GSI XVI), N-[N-3,5- Difluorophenacetyl]-L- alanyl-S-phenylglycine Methyl Ester; γ- secretase inhibitor XVII (GSI XVII); γ-secretase inhibitor XIX (GSI XIX), benzo[e][1,4]diazepin-3-yl)-butyramide; γ-secretase inhibitor XX (GSI XX), (S,S)-2-[2-(3,5- Difluorophenyl)acetylamino]- N-(5-methyl-6-oxo-6,7- dihydro-5H-dibenzo[b,d]azepin-7- yl)propionamide; γ-secretase inhibitor XXI (GSI XXI), (S,S)-2-[2-(3,5-Difluorophenyl)-acetylamino]-N-(1-methyl-2-oxo-5-phenyl-2-,3-dihydro-1H-benzo[e][1,4]diazepin-3-yl)-propionamide; Gamma40 secretase inhibitor I, N-trans-3,5-Dimethoxycinnamoyl-Ile- leucinal; Gamma40 secretase inhibitor II, N-tert-Butyloxycarbonyl-Gly-Val-Valinal Isovaleryl-V V-Sta-A-Sta- OCH3; MK-0752 (Merck); MRK-003 (Merck); semagacestat/LY450139 (Eli Lilly); RO4929097; PF-03084,014; BMS-708163; MPC-7869 (γ-secretase modifier), YO-01027 (Dibenzazepine), Compound E ([(2S)-2-{[(3,5-Difluorophenyl)acetyl]amino}-N-[(3S)-1-methyl-2-oxo-5-phenyl-2,3-dihydro-1H-1,4-benzodiazepin-3-yl]propanamide], available from Alexis Biochemicals), LY411575 (Eli Lilly and Co.), L-685,458 (Sigma-Aldrich), BMS-289948 (4-chloro-N-(2,5-difluorophenyl)-N-((1R)-{4-fluoro-2-[3-(1H-imidazol-1-yl)propyl]phenyl}ethyl)benzenesulfonamide hydrochloride) and BMS-299897 (4-[2-((1R)-1-{[(4-chlorophenyl)sulfonyl]-2,5-difluoroanilino}ethyl)-5-fluorophenyl]butanoic acid) (Bristol Myers Squibb).
15. The method according to any one of the preceding items, wherein the gamma secretase modulator is intravenously administered to the subject.
16. The method according to any one of the preceding items, wherein the gamma secretase modulator is orally administered to the subject.
17. The method according to any one of the preceding items, wherein the subject is being treated with or has been previously treated with radiation therapy, chemotherapy, transplantation, immunotherapy, hormone therapy, or photodynamic therapy.
18. The method according to any one of the preceding items, wherein the subject is being treated with or has been previously treated for a B cell condition or disorder that targets BCMA on B cells.
19. The method according to any one of the preceding items, wherein the subject has a refractory hematological malignancy.

## Claims

1. A therapeutic agent that binds to BCMA for use in treating or preventing a B cell condition or disorder in a subject, wherein the use comprises co-administering of said therapeutic agent that binds to BCMA together with one or more gamma secretase inhibitor(s) to the subject.

2. One or more gamma secretase inhibitor(s) or a derivative thereof for use in a method of improving the efficacy of a therapeutic agent that binds to BCMA in the treatment of a B cell condition or disorder, wherein the therapeutic agent that binds to BCMA is selected from the group consisting of a chimeric antigen receptor T cell (CAR-T cell) that bind to BCMA, a therapeutic anti-BCMA antibody or a fragment thereof and an anti-BCMA antibody-drug conjugate.

3. The therapeutic agent for use of claim 1 or the one or more gamma secretase inhibitor(s) or the derivative thereof for use of claim 2, wherein the B cell condition or disorder is selected from the group consisting of: MM, CLL, WM, B cell non-Hodgkin's lymphoma, plasmacytoma, Hodgkins' lymphoma, follicular lymphomas, small non-cleaved cell lymphomas, endemic Burkitt's lymphoma, sporadic Burkitt's lymphoma, marginal zone lymphoma, extranodal mucosa-associated lymphoid tissue lymphoma, nodal monocytoid B cell lymphoma, splenic lymphoma, mantle cell lymphoma, large cell lymphoma, diffuse mixed cell lymphoma, immunoblastic lymphoma, primary mediastinal B cell lymphoma, pulmonary B cell angiocentric lymphoma, small lymphocytic lymphoma, B cell proliferations of uncertain malignant potential, lymphomatoid granulomatosis, post-transplant lymphoproliferative disorder, an immunoregulatory disorder, rheumatoid arthritis, myasthenia gravis, idiopathic thrombocytopenia purpura, anti-phospholipid syndrome, Chagas' disease, Grave's disease, Wegener's granulomatosis, poly-arteritis nodosa, Sjogren's syndrome, pemphigus vulgaris, scleroderma, multiple sclerosis, anti-phospholipid syndrome, ANCA associated vasculitis, Goodpasture's disease, Kawasaki disease, autoimmune hemolytic anemia, and rapidly progressive glomerulonephritis, heavy-chain disease, primary or immunocyte-associated amyloidosis, and monoclonal gammopathy of undetermined significance.

4. The therapeutic agent for use of claim 1 or the one or more gamma secretase inhibitor(s) or the derivative thereof for use of claim 2, wherein the B cell condition or disorder is a B cell malignancy.

5. The therapeutic agent for use of claim 1 or the one or more gamma secretase inhibitor(s) or the derivative thereof for use of claim 2, wherein the B cell condition or disorder is a plasma cell malignancy.

6. The therapeutic agent for use of claim 1 or the one or more gamma secretase inhibitor(s) or the derivative thereof for use of claim 2, wherein the B cell condition or disorder is selected from the group consisting of: MM, WM, CLL, and B-cell non-Hodgkin's lymphoma.

7. The therapeutic agent for use of claim 1 or the one or more gamma secretase inhibitor(s) or the derivative thereof for use of claim 2, wherein the B cell condition or disorder is multiple myeloma.

8. The therapeutic agent for use of claim 1 or the one or more gamma secretase inhibitor(s) or the derivative thereof for use of claim 2, wherein the B cell condition or disorder is an autoimmune disease.

9. The therapeutic agent for use of claim 1 or the one or more gamma secretase inhibitor(s) or the derivative thereof for use of claim 2, wherein the B cell condition or disorder is systemic lupus erythematosus.

10. The therapeutic agent for use of claim 1 or the one or more gamma secretase inhibitor(s) or the derivative thereof for use of claim 2, wherein the B cell condition or disorder is rheumatoid arthritis.

11. The therapeutic agent for use of claim 1 or the one or more gamma secretase inhibitor(s) or the derivative thereof for use of claim 2, wherein the B cell condition or disorder is selected from the group consisting of: idiopathic thrombocytopenia purpura, myasthenia gravis, and autoimmune hemolytic anemia.

12. The therapeutic agent for use of any one of claims 1 and 3-11, or the one or more gamma secretase inhibitor(s) or the derivative thereof for use of any one of claims 2-11, wherein the gamma secretase inhibitor is selected from the group consisting of: PF-03084014, MK-0752, MRK-003, LY450139, RO4929097, BMS-708163, MPC-7869, LY411575, L-685458, Z-Leu-Leu-Norleucine-CHO, N-Benzyloxycarbonyl-Leu-leucinal, N-(2-Naphthoyl)-Val-phenylalaninal, N-Benzyloxycarbonyl-Leu-phenylalaninal, I-(S)-endo-N-(1,3,3)-Trimethylbicyclo[2.2.1]hept-2-yl)-4-fluorophenyl Sulfonamide, Menthyloxycarbonyl-LL-CHO, N-[N-(3,5-Difluorophenacetyl-L-alanyl)]-S-phenylglycine t-Butyl Ester, {IS-Benzyl-4R-[I-(IS-carbamoyl-2-phenethylcarbamoyl)-IS-3-methylbutylcarb-amoyl]-2R-hydroxy-5-phenylpentyl}carbamic Acid tert-butyl Ester, 7-Amino-4-chloro-3-methoxyisocoumarin, Z-Ile-Leu-CHO, Z-Tyr-Ile-Leu-CHO, Z-Cys(t-Bu)-Ile-Leu-CHO, N-[N-3,5-Difluorophenacetyl]-L-alanyl-S-phenylglycine Methyl Ester, benzo[e][I,4]diazepin-3-yl)-butyramide, (S,S)-2-[2-(3,5-Difluorophenyl)acetylamino]-N-(5-methyl-6-oxo-6,7-dihydro-5H-dibenzo[b,d]azepin-7-yl)propionamide, (S,S)-2-[2-(3,5-Difluorophenyl)-acetylamino]-N-(I-methyl-2-oxo-5-phenyl-2-,3-dihydro-IH-benzo[e][I,4]diazepin-3-yl)-propionamide, N-trans-3,5-Dimethoxycinnamoyl-Ile-leucinal, N-tert-Butyloxycarbonyl-Gly-Val-Valinal, Isovaleryl-V-V-Sta-A-Sta-OCH3, Dibenzazepine, [(2S)-2-{[(3,5-Difluorophenyl)acetyl]amino}-N-[(3S)-1-methyl-2-oxo-5-phenyl-2,3-dihydro-1H-1,4-benzodiazepin-3-yl]propanamide]; and 4-chloro-N-(2,5-difluorophenyl)-N-((1R)-{4-fluoro-2-[3-(1H-imidazol-1-yl)propyl]phenyl}ethyl)benzenesulfonamide.

13. The therapeutic agent for use of any one of claims 1 and 3-12, or the one or more gamma secretase inhibitor(s) or the derivative thereof for use of any one of claims 2-12, wherein the gamma secretase inhibitor is intravenously administered to the subject

14. The therapeutic agent for use of any one of claims 1 and 3-13, or the one or more gamma secretase inhibitor(s) or the derivative thereof for use of any one of claims 2-13, wherein the gamma secretase inhibitor is orally administered to the subject.

15. The therapeutic agent for use of any one of claims 1 and 3-14, or the one or more gamma secretase inhibitor(s) or the derivative thereof for use of any one of claims 2-14, wherein the subject is being treated with or has been previously treated with radiation therapy, chemotherapy, transplantation, immunotherapy, hormone therapy, or photodynamic therapy.
